# EUROPEAN PATENT SPECIFICATION

(11) **EP 0 371 509 B1**
(45) Date of publication and mention of the grant of the patent: **01.02.1995**
(21) Application number: 89122134.3
(22) Date of filing: 30.11.1989
(51) Int. Cl.: C07G 11/00, C12P 1/06, C12N 1/20, A61K 35/66

(54) **Antibiotics and process for producing them**
Antibiotika und deren Verfahren zur Herstellung
Antibiotiques et leur procédé de préparation

(30) Priority: 01.12.1988 IT 2280888
(43) Date of publication of application: 06.06.1990
(73) Proprietor: MINISTERO DELL' UNIVERSITA' E DELLA RICERCA SCIENTIFICA E TECNOLOGICA, 00196 Roma (IT)
(72) Inventor: Cidaria, Dante, I-28100 Novara (IT); Andriollo, Nunzio, I-20021 Bollate Milan (IT); Cassani, Giorgio, I-20010 Arluno Milan (IT); Crestani, Enrico, I-28100 Novara (IT); Spera, Silvia, I-21020 Daverio Varese (IT); Garavaglia, Carlo, I-20012 Cuggiono Milan (IT); Pirali, Giorgio, I-21047 Saronno Varese (IT); Confalonieri, Giovanni, I-20052 Monza Milan (IT)
(74) Representative: Weinhold, Peter, Dr.

## Description

The present invention relates to antibiotic substances named "AB-021 Antibiotics" and to the main components thereof, i.e. antibiotic AB-021 a and antibiotic AB-021 b.

Furthermore, the present invention relates to the process for preparing said antibiotics by means of the fermentation of Streptomyces s p. NCIB 40068, and to their use in the treatment of the infective diseases caused by microorganisms susceptible to said antibiotics.

The AB-021 antibiotics, as well as the components thereof, i.e. antibiotic AB-021 a and antibiotic AB-021 b, are different from the known antibiotics.

The term "AB-021 Antibiotics" used in the present application is meant to denote a mixture which comprises all of the components endowed with biological activity such as, for example, antifungal and/or antibacterial activity, produced by the fermentation of Streptomyces s p. NCIB 40068 under the conditions specified in the following.

Said biologically active components comprise, but are not limited to, those designated as AB-021 a and AB-021 b, which can be isolated from the mixture.

It is self-evident that the number and the mutual ratios of the components which constitute the AB-021 antibiotics may vary as a function of the fermentation conditions (such as, e.g., the culture medium, fermentation temperature, the duration of the fermentation, the aeration), and of the bacterial strain used.

Furthermore, it is to be understood that the present invention is not limited to the use of Streptomyces s p. NCIB 40068, but also comprises the use of variants and/or mutants spontaneously or artificially obtained from the above microorganism, provided that they produce the AB-021 antibiotics.

Therefore, the present invention is directed to the AB-021 antibiotics obtainable by means of the controlled cultivation under aerobic conditions of Streptomyces s p. NCIB 40068, or of an equivalent spontaneous or artificial variant or mutant thereof, in an aqueous nutrient cultivation medium, containing sources of carbon and nitrogen as well as inorganic salts and metals in trace amounts, and subsequent separation of said antibiotics and of the main components thereof, i.e. antibiotics AB-021 a and AB-021 b.

### Physico-Chemical Characteristics of antibiotic AB-021 a

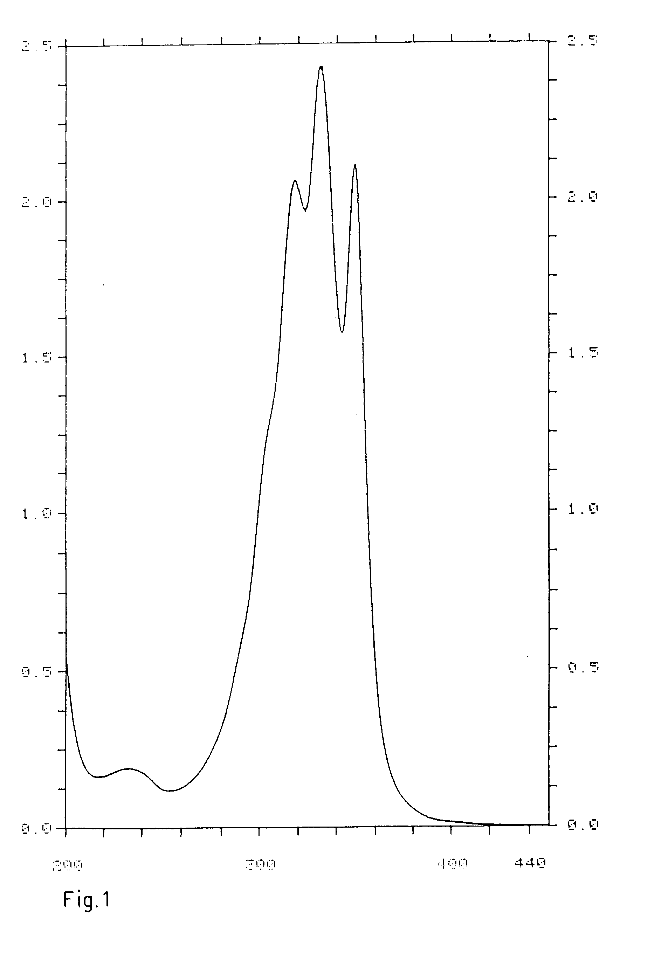

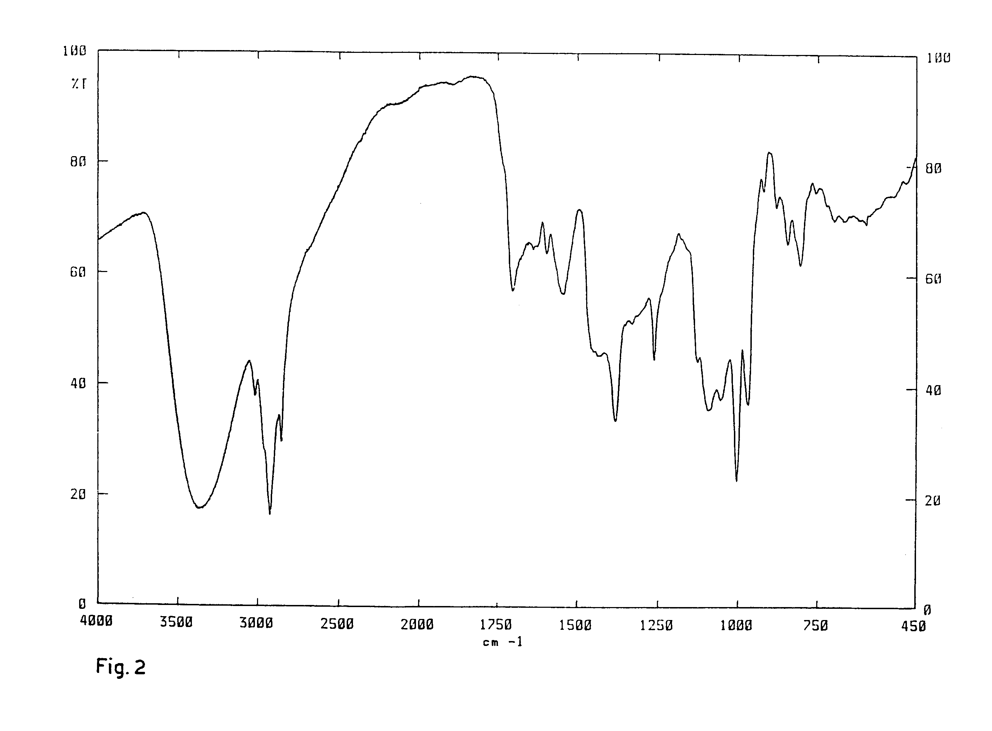

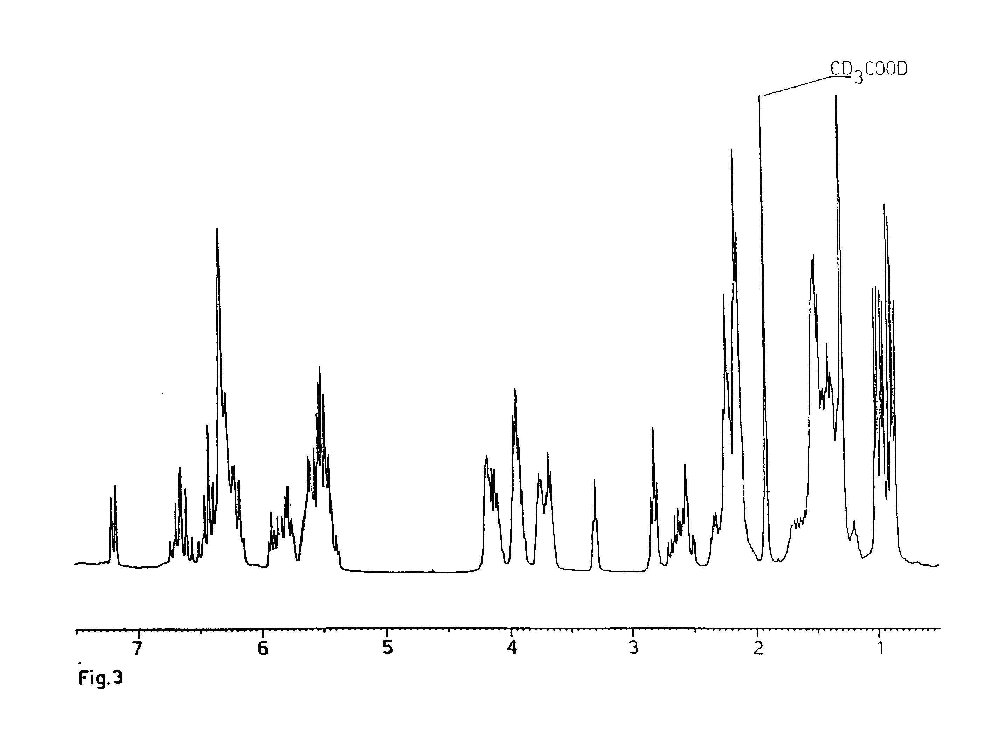

AB-021 a, a component of the AB-021 antibiotics, is a powder of light yellow colour, characterized by:
(a) an approximate elemental analysis, determined on a sample left to stand under vacuum for 2 hours at 40 _{°} C as follows: 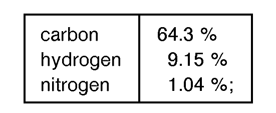 said analysis revealing neither sulfur nor phosphorus;
(b) a molecular weight of about 1139, as determined by FAB-MS spectroscopy, yielding a peak at 1138, corresponding to (M-H)-, under the following operating conditions:
   - Negative ions, FAB, Xe at 9.5 kV
   - Matrix: glycerol
   - Finnigan Mat 8428;
(c) an U.V. absorption spectrum as shown in Figure 1 of the attached drawings. Said spectrum shows absorbance maxima of 0.193 at 232.8 nm; 2.064 at 318.4 nm; 2.44 at 332.6 nm; and 2.113 at 349.6 nm; determined at a concentration of 0.025 mg/ml in 1:1 (V/V) acetonitrile: water;
(d) an infrared (I.R.) absorption spectrum in KBr pellet as shown in Figure 2 of the attached drawings, exhibiting the following absorption maxima (cm-¹): 3365; 3015; 2923; 2851; 1700; 1634; 1594; 1541; 1431; 1380; 1329; 1262; 1128; 1095; 1056; 1004; 968; 919; 878; 843; 804; 754;
(e) an 'H-NMR-spectrum recorded on a BRUKER AM 300 MHz spectrometer in hexa-deutero-dimethylsulfoxide (DMSO-d6) to which very small amounts of tetradeuterated acetic acid had been added and shown in Figure 3, which spectrum exhibits the following signals:

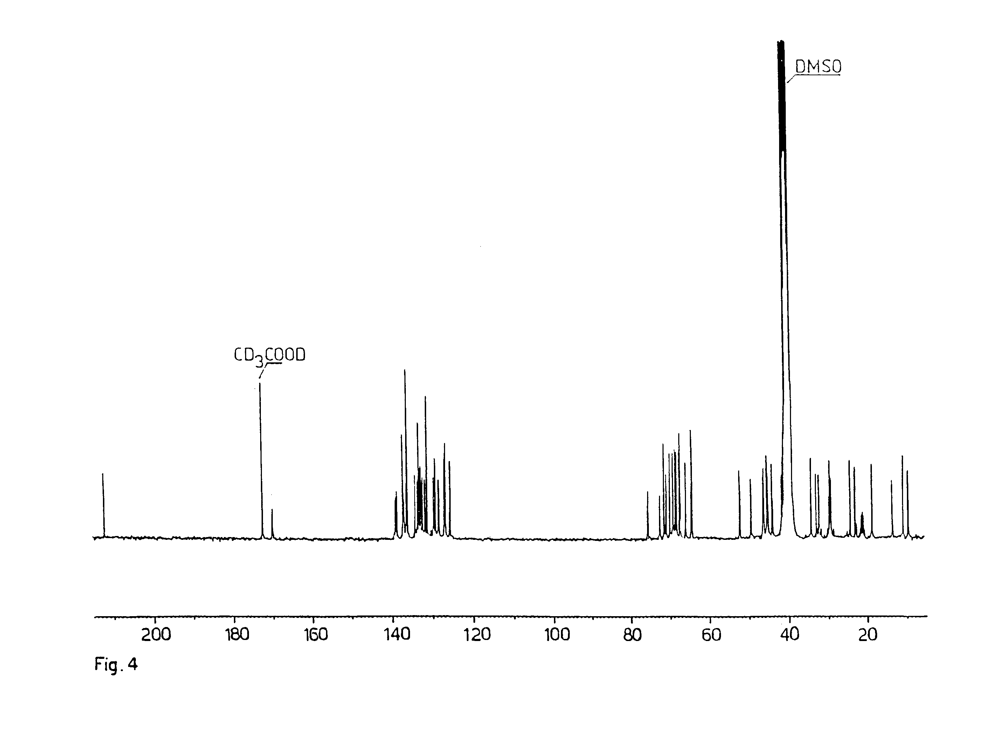

(The chemical shifts were indirectly based on TMS = 0.00 ppm (STMS) by using the central peak of hexa-deuterodimethylsulfoxide at 6TMS = 2.56 ppm as internal reference.)
6TMS (ppm) : 7.18 (d, 1 H); 6.69 (dd, 1 H); 6.59 (dd, 1 H); 6.52 - 6.09 (m, 9H); 5.96 - 5.70 (m, 3H); 5.70 - 5.31 (m, 8H); 4.27 - 4.02 (m, 4H); 4.02 - 3.82 (m, 4H); 3.82 - 3.58 (m, 4H); 3.30 (t, 1 H); 2.82 (t, 2H); 2.73 - 2.45 (^{*}) (m, 3H); 2.41 - 1.98 (m, 13H); 1.91 (s, 3H ); 1.79 - 1.12 (m, 24-25H), 1.00 (d, 3H; 0.95 (d, 3H); 0.89 (d, 3H); 0.85 (d, 3H);
(f) a ¹³C-NMR spectrum recorded on a BRUKER AM 300 MHz spectrometer in hexa-deutero-dimethylsulfoxide (DMSO-d6) to which very small amounts of tetradeuterated acetic acid had been added and shown in Figure 4, which spectrum exhibits the following signals:
   (The chemical shifts were indirectly based on TMS = 0.00 ppm (δTMS) by using the central peak of hexa-deuterodimethylsulfoxide at 6TMS = 39.85 ppm as internal reference; the data concerning the multiplicity of the signals were obtained by means of DEPT tests at 45°, 90 ° and 135°.)
   6TMS (ppm) : 212.1 (s); 169.9 (s); 138.8 (d); 138.5 (d); 137.0 (d); 136.9 (d); 136.7 (d); 136.1 (d); 135.9 (d); 135.9 (d); 134.0 (d); 133.3 (d); 133.0 (d), 132.9 (d); 132.5 (d); 132.4 (d); 132.0 (d), 131.5 (d); 131.0 (d); 131.0 (d); 129.3 (d), 128.9 (d); 128.0 (d); 126.7 (d); 126.4 (d); 125.3 (d); 75.3 (d); 72.3 (d); 71.2 (d); 70.6 (d); 69.7 (d); 68.9 (d); 68.4 (d); 68.0 (d); 67.1 (d); 67.1 (d); 65.7 (d); 64.2 (d); 64.2 (d); 52.1 (d); 41.5 (d); 39.9 (d); 49.2 (t); 46.2 (t); 46.0 (t); 45.2 (t); 45.2 (t); 44.9 (t); 43.9 (t); 41.2 (t); 40.8 (t); 40.7 (t); 39.2 (t); 38.8 (t); 34.1 (t); 32.8 (t); 32.1 (t); 29.3 (t); 24.1 (t); 22.8 (q); 18.4 (q); 13.2 (q); 10.5 (q); 9.1 (q).
(g) retention coefficients in thin-layer chromatography on silica (Kieselgel) plates of the type 60 F 254 (Merck-Schuchardt) and on reverse-phase silica plates of the type RP-18F 254 (Merck-Schuchardt) in the following eluent systems and compared to antibiotic AB-021 b (distance covered by eluent: 15 cm):
   eluent A : methanol : acetonitrile : 25 mM monohydrogen sodium phosphate in water (4:4:2);
   eluent B : methanol : acetonitrile : 25 mM dihydrogen potassium phosphate + 7 mM tetramethylammonium chloride in water (4:4:2);
   eluent C : methanol : 10 mM monohydrogen ammonium phosphate in water (adjusted to pH 7.5 with phosphoric acid) (8:2);
   eluent D : methanol : acetonitrile : 10 mM monohydrogen ammonium phosphate in water(adjusted to pH 7.5 with phosphoric acid)(4:4:2);
   eluent E : ethanol : dioxane : aqueous solution of ammonia (30%) : water (8:1:1:1);
   eluent F : methylene chloride : methanol (17:3).

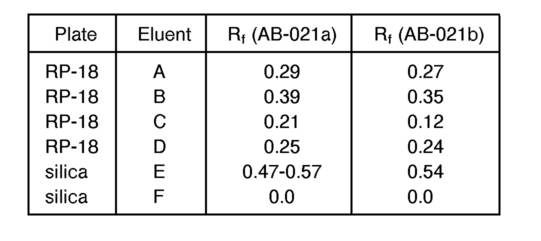

### Visualization:

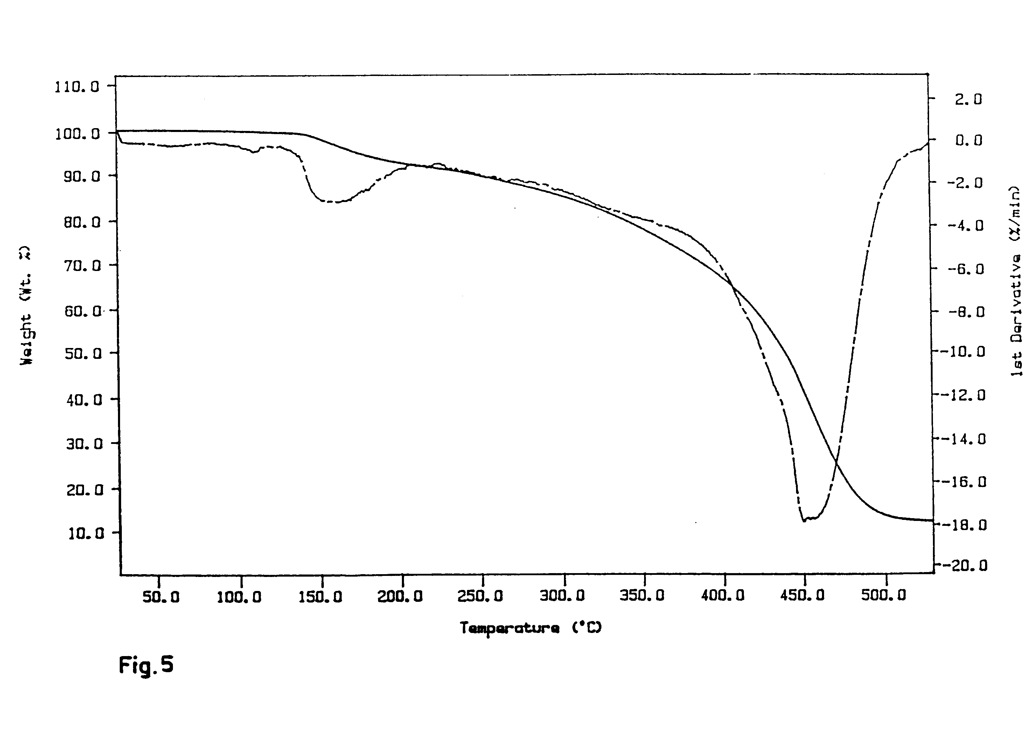

A. Fluorescence in U.V. light (366 nm)
B. Anisaldehyde (T-27 reactant) - Thin Layer Chromatography - page 205 Author: Justus G. Kirchner - 2nd Ed. Publisher: John Wiley & Sons.
C. o-Aminophenol (T-11 reactant) - Thin Layer Chromatography - page 201 Author: Justus G. Kirchner - 2nd Ed. Publisher: John Wiley & Sons.
(h) a retention time (Rₜ) of about 8 minutes when subjected to reverse-phase HPLC column chromatography under the following conditions:
   Column : Hibar LichroCARTO Li-Chrosorb RP-18 (7 µm) 260 x 4.0 mm (Merck, Darmstadt, F.R. of Germany)
   Forecolumn : Guard Pak@ RCSS C 18 (Millipore Waters)
   Eluent : methanol : acetonitrile : 10 mM monohydrogen ammonium phosphate in water (1:1:1)
   (^{*}) within this range, also the peak of DMSO-d6 appears.
   Flow rate : 0.8 ml/minute
   Detector : U.V. at 333 nm
   Temperature : 40 _{°} C (Under the same conditions antibiotic AB-021 b is eluted after about 11.8 minutes.)
(i) a good solubility in dimethylsulfoxide and in (1:1 V/V) ethanol/water or (1:1 V/V) methanol/water mixtures [V/V = volume/volume], poor solubility in water, fairly good solubility in ethanol and methanol;
(j) a diagram derived from a thermogravimetric analysis carried out under nitrogen, with a temperature increase rate of 20 ° C/minute within the temperature range of from 30 ° C up to 700 ° C on a PERKIN-ELMER 7 SERIES Thermal Analysis System, said plot being shown in Figure 5, in which on the abscissa the temperature is given in ° C, and on the ordinate the percent weight loss is indicated. In said Figure 5 also the first derivative of the curve is shown.

### Physico-Chemical Characteristics of Antibiotic AB-021 b

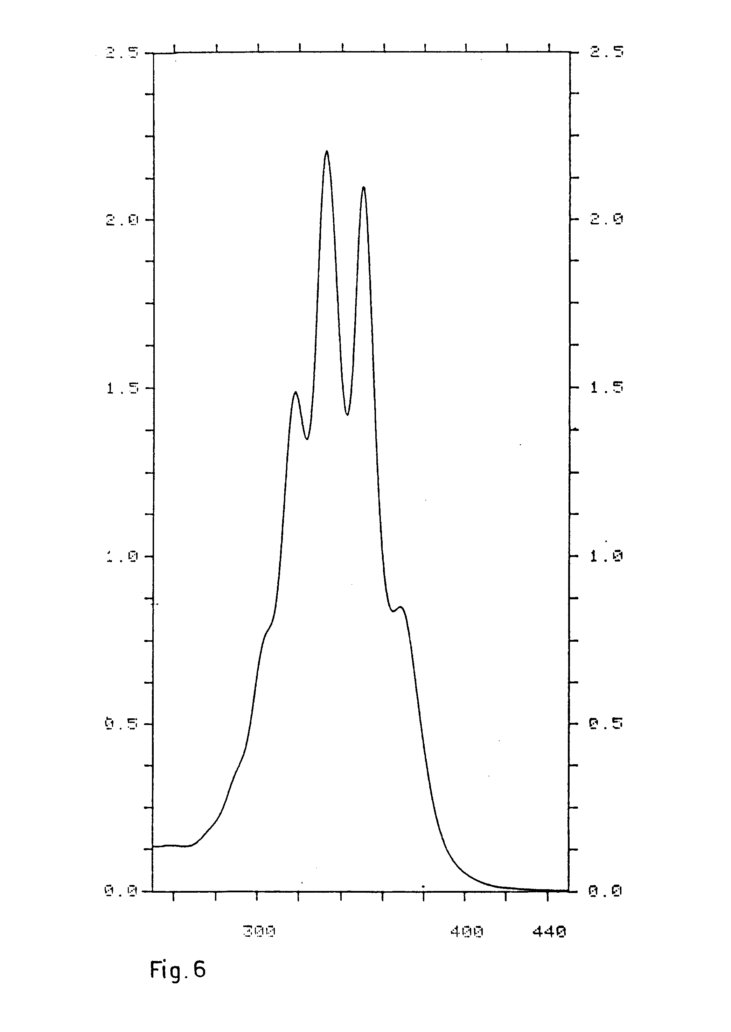

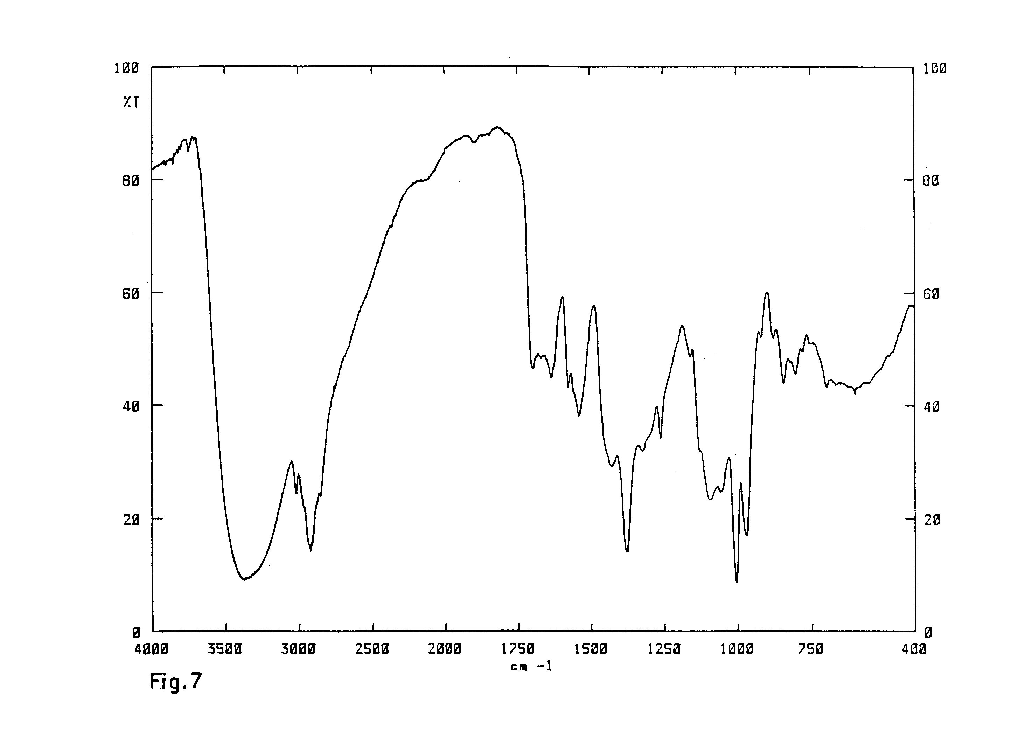

AB-021 b, a component of the AB-021 antibiotics, is a powder of deep yellow colour, characterized by:
(a) an approximate elemental analysis, determined on a sample left to stand under vacuum at 40 ° C for 2 hours, as follows: 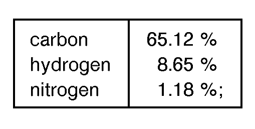
(b) a molecular weight of about 1165 as determined by FAB-MS spectroscopy, affording a peak at 1164, corresponding to (M-H)-, under the following operating conditions:
   - Negative ions, FAB, Xe at 9.5 kV
   - Matrix: glycerol
   - Finnigan Mat 8424;
(c) the U.V. absorption spectrum shown in Figure 6 of the attached drawings. Said spectrum shows the following absorbance maxima: 0.90 at 369.0 nm; 2.01 at 350.0 nm; 2.21 at 332.6 nm; 1.49 at 318.0 nm; measured at a concentration of 0.02 mg/ml in 1 : 1 (V/V) acetonitrile/water;
(d) an infrared (I.R.) absorption spectrum (KBr pellet) as shown in Figure 7 of the attached drawings, exhibiting the following absorption maxima (cm-¹):

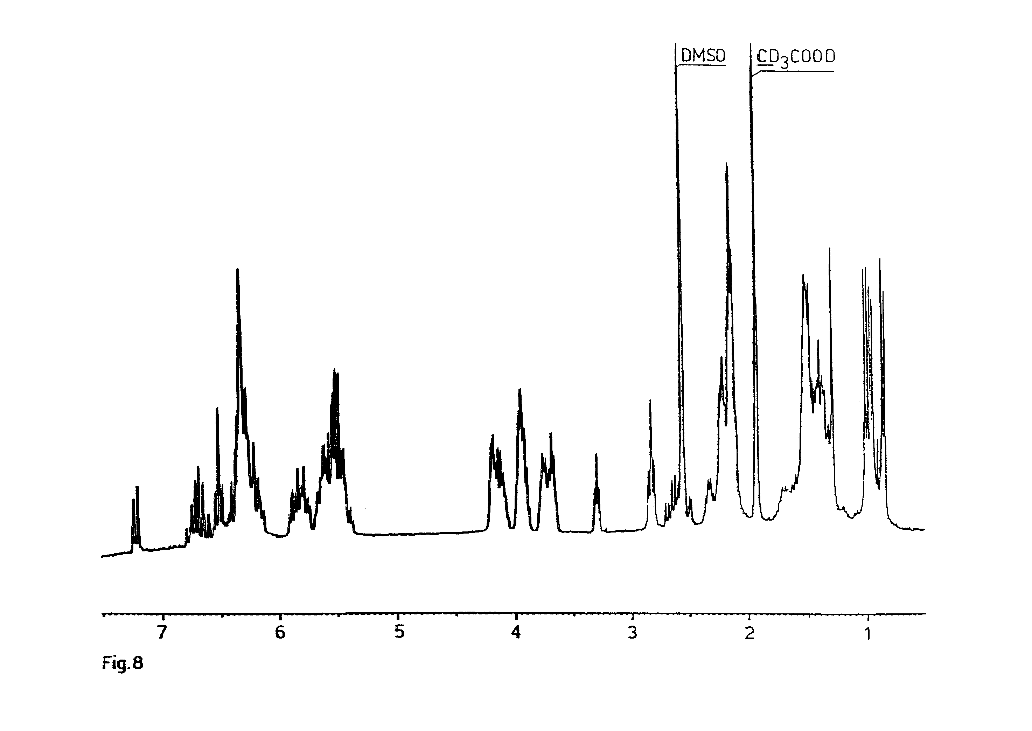

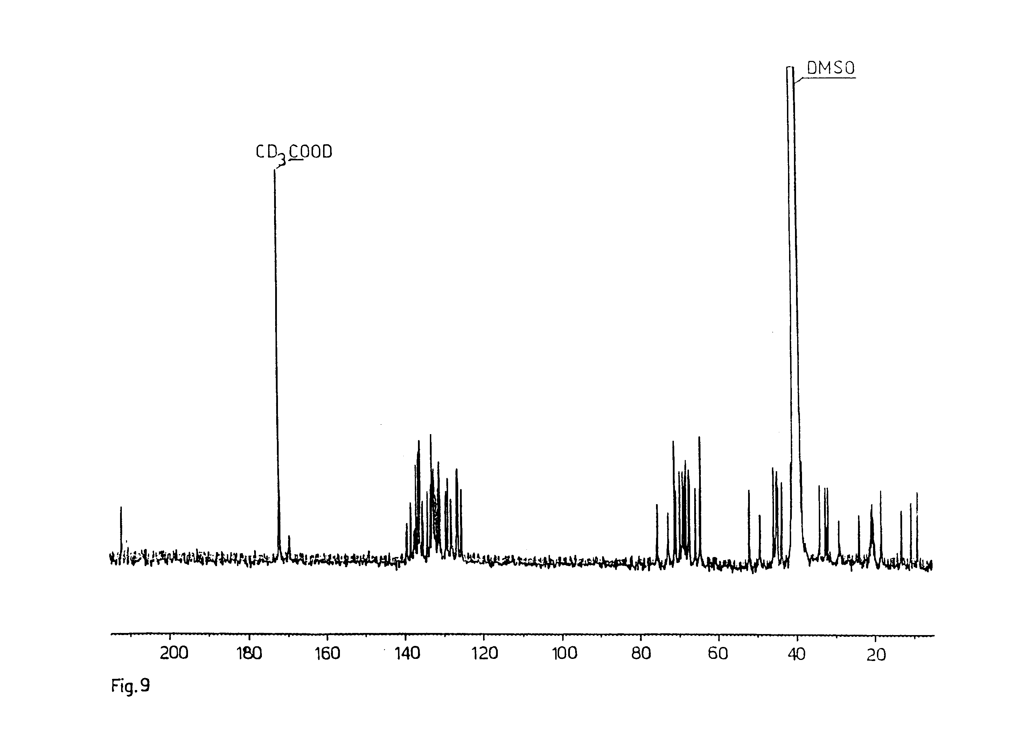

3902, 3853, 3747, 3375, 3013, 2921, 2853, 2757, 1895, 1699, 1669, 1645, 1576, 1540, 1430, 1378, 1324, 1261, 1160, 1094, 1059, 1004, 969, 919, 878, 843, 803, 779, 697;
(e) a ¹H-NMR spectrum recorded on a BRUKER AM 300 MHz spectrometer in hexa-deutero-dimethylsulfoxide (DMSO-d6) to which small amounts of tetradeuterated acetic acid had been added and shown in Figure 8 of the attached drawings, said spectrum exhibiting the following signals: (The chemical shifts were indirectly based on TMS = 0.00 ppm (δTMS) by using the central peak of hexa-deuterodimethylsulfoxide at 6TMS = 2.56 ppm as internal reference.)
   δTMS (ppm) : 7.21 (d, 1 H); 6.75 (dd, 1 H), 6.63 (dd, 1 H); 6.57 - 6.07 (m, 11 H); 5.93 - 5.70 (m, 3H); 5.70 - 5.33 (m, 8H); 4.25 - 4.02 (m, 4H); 4.02 - 3.83 (m, 4H), 3.83 - 3.59 (m, 4H); 3.29 (t, 1 H); 2.82 (t, 2H); 2.72 - 2.44 (^{*}) (m, 6-7H); 2.41 - 2.00 (m, 14H); 1.92 (s, 3H), 1.80 - 1.14 (m, 21 H); 1.00 (d, 3H); 0.95 (d, 3H), 0.84 (d, 3H).
(f) a ¹³C-NMR spectrum recorded on a BRUKER AM 300 MHz spectrometer in hexa-deutero-dimethylsulfoxide (DMSO-d6) and shown in Figure 9 of the attached drawings, said spectrum exhibiting the following signals:

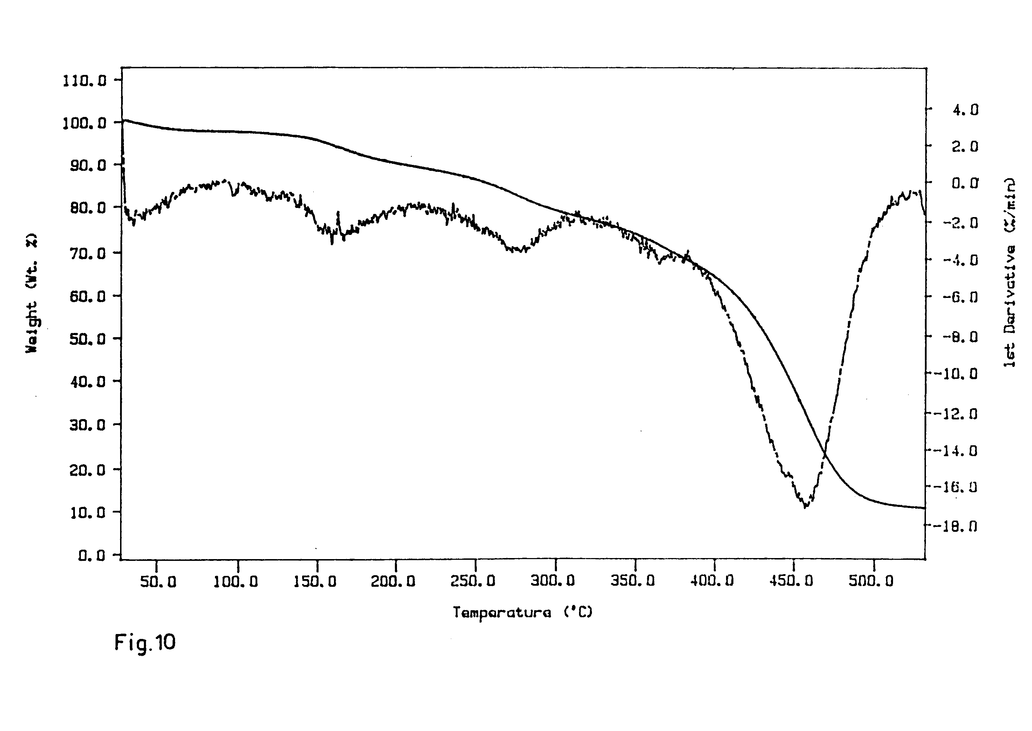

(The chemical shifts were indirectly based on TMS = 0.00 ppm (δTMS) by using the central peak of hexa-deuterodimethylsulfoxide at δTMS = 39.85 ppm as internal reference. The data concerning the multiplicity of the signals were obtained by means of DEPT tests at 45 ° , 90 ° and 135 °.)
δTMS (ppm) : 212.0 (s); 169.2 (s); 139.1 (d); 138.1 (d); 137.1 (d); 136.7 (d); 136.2 (d); 135.8 (d); 135.8 (d); 135.1 (d); 133.8 (d); 133.1 (d); 132.8 (d); 132.8 (d); 132.6 (d); 132.3 (d); 132.2 (d); 132.1 (d); 131.8 (d); 131.2 (d); 130.9 (d); 130.8 (d); 129.2 (d); 128.7 (d); 127.9 (d); 126.4 (d); 126.2 (d); 125.2 (d); 75.4 (d); 72.6 (d); 71.0 (d); 70.6 (d); 69.6 (d); 68.9 (d); 68.4 (d); 68.0 (d); 67.3 (d); 67.2 (d); 65.6 (d); 64.4 (d); 64.4 (d); 51.9 (d); 41.3 (d); 39.6 (d); 49.2 (t), 45.8 (t), 45.7 (t); 45.0 (t); 44.9 (t); 44.7 (t), 43.6 (t); 41.1 (t);
(^{*}) Within this range also the peak of DMSO-d6 appears. 40.7 (t); 40.5 (t); 39.0 (t); 38.7 (t); 33.9 (t); 32.5 (t); 31.9 (t); 29.0 (t); 23.9 (t); 18.1 (q); 12.8 (q); 10.4 (q); 8.7 (q).
(g) the retention coefficients (R_{f}) in thin-layer chromatography and the retention times (Rₜ) on a reverse-phase HPLC column, respectively, as reported under paragraphs (g) and (h) of the description of the physico-chemical characteristics of antibiotic AB-021 a.
(h) a diagram derived from a thermogravimetric analysis carried out under nitrogen, with a temperature increase rate of 20 ° C/minute within the temperature range of from 30 ° C up to 700 °C on a PERKIN-ELMER 7 SERIES Thermal Analysis System, said plot being shown in Figure 10, in which on the abscissa the temperature is given in ° C, and on the ordinate the percent weight loss is indicated. In said Figure 10 also the first derivative of the curve is shown.

### Morphology and culture characteristics of microorganism Streptomyces s p. NCIB 40068

The microorganism Streptomyces s p. NCIB 40068 was isolated from a sample of soil collected at Cappella di Terza (Novara), and catalogued for internal laboratory use under the designation SD-505 W.

A culture of this microorganism was deposited on September 29th 1988, in compliance with the Budapest Treaty, with the National Collection of Industrial Bacteria (c/o the National Collection of Industrial and Marine Bacteria Ltd., Torry Research Station, P.O. Box 31, 135 Abbey Road, Aberdeen AB 98 DG, Scotland, United Kingdom), where it was given the access number NCIB 40068.

The morphological characteristics of the strain are listed in Table A (the names of the culture media are those assigned by the International Streptomyces Program).

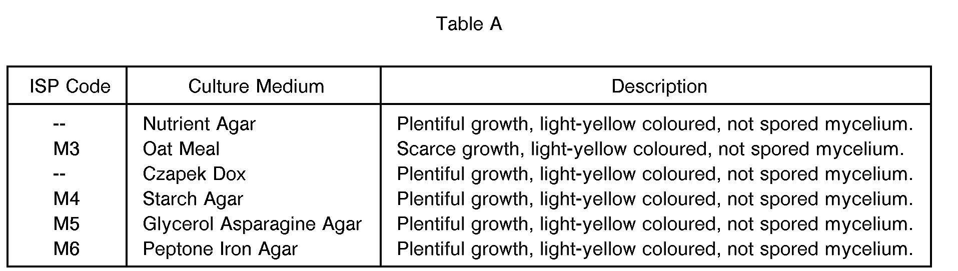

In Tables B and C, some characteristics of this strain are reported.

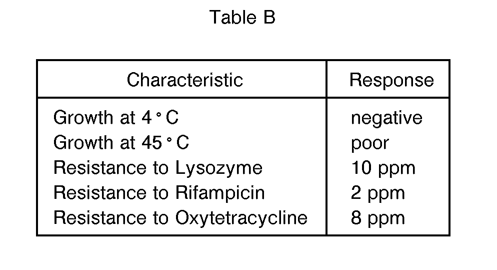

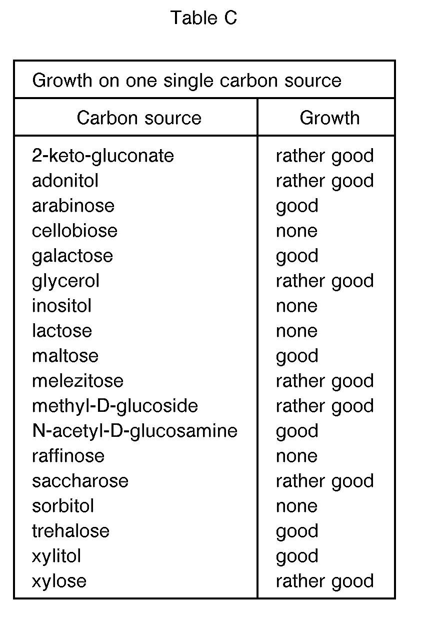

The analysis of the cellular wall of Streptomyces sp. NCIB 40068 was carried out according to the method described by M.P. Starr, H. Stolp, H.G. Truper, A. Ballows, H.G. Shegel in "The Prokaryotes" - Vol. II Streptomycetacee - Springer Verlag Ed., 1981). Said analysis demonstrates the absence of characteristic sugars; thus it confirms that the strain SD-505W belongs to the Streptomyces genus.

Like other microorganisms; Streptomyces s p. NCIB 40068 can undergo variations and/or mutations of either spontaneous or artificial character.

For example, artificial variants or mutants can be obtained by treating the microorganism with chemical mutagenic agents, such as, e.g., nitrous acid, nitroso-guanidine, halogenated alkylamines and the like, or with physical agents, such as X-rays or U.V. light and high-frequency waves.

Spontaneous variants or mutants can be obtained as well by isolating and selecting different colonies obtained from Streptomyces s p. NCIB 40068.

All of the variants and/or mutants, both of natural origin or man-made, which belong to the species Streptomyces and produce AB-021 antibiotics are considered to be equivalent to the Streptomyces s p. strain NCIB 40068 microorganism and are comprised within the scope of the present invention.

### Process of preparation of AB-021 Antibiotics

The process for the preparation of AB-021 antibiotics comprises the cultivation of Streptomyces s p. NCIB 40068 or an equivalent (spontaneous or man-made) variant or mutant thereof under conditions of controlled aerobic fermentation in an aqueous nutrient medium, and the subsequent separation of said AB-021 antibiotic by means of per se known methods.

As nutrient culture media or fermentation broths those which are commonly used for the production of antibiotics can be used; however, some nutrient culture media are preferred.

Said cultivation media should contain sources of carbon and nitrogen which can be assimilated by the microorganisms of the genus Streptomyces and should furthermore show low levels of inorganic salts.

Moreover, they should contain traces of those metals which are necessary for the growth and the development of the microorganisms and for the production of the antibiotics. Said metal traces can already be present as impurities in the sources of carbon and of organic nitrogen provided in the culture media for the bacterial growth or, optionally, they can be added to the culture media.

As carbon source there can generally be used carbohydrates, in particular saccharides, such as, e.g., dextrose or maltose or, as an alternative and/or complement, starches and products which are considered similar to starches from an industrial point of view, such as, e.g., dextrin or soluble starch, or also polyalcohols, such as, e.g., glycerol.

Said compounds can be used individually or combined with one another in variable proportions.

The concentration and type of the carbon source in the culture medium generally depend on the type and amount of the other ingredients contained in said medium; anyway, concentrations of from 0.5 to 5% by weight generally lead to satisfactory results.

As the source of organic nitrogen, both proteinic extracts such as, e.g., yeast extract, meat extract or casein hydrolysate, or meals such as, e.g., soybean meal, or commercially available products for that purpose such as, e.g., Proflo, Corn Steep Liquor or Distillers' Solubles, can be used.

These products can be used individually or combined with one another in variable proportions. The concentrations in the culture medium generally range from 0.2 % to 6 % by weight.

As inorganic salts there can be used, for example, sodium salts, potassium salts, magnesium salts, ammonium salts and calcium salts, such as phosphates, sulfates, chlorides, carbonates and nitrates.

Examples of the trace metals contained in the culture medium are cobalt, iron, manganese and the like.

Some culture media display a particular ability to stimulate the production of AB-021 antibiotics by Streptomyces s p. NCIB 40068; among these,for example, the following aqueous formulations can be mentioned, which have been used in the following preparation Examples.

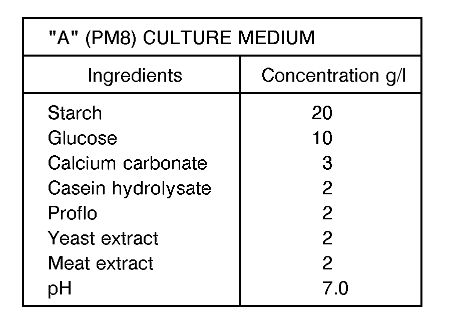

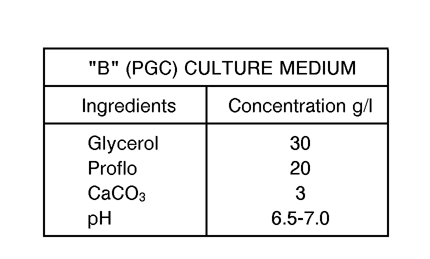

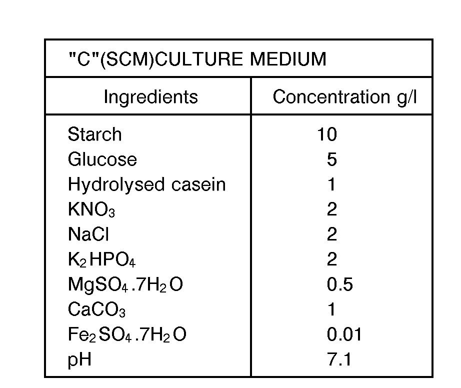

The strain of Streptomyces s p. NCIB 40068 can be cultivated and fermented at temperatures of from 20 ° C to 35 ° C, preferably of from 25 ° C to 30 ° C.

The pH value generally is within the range of from about 5 to about 9.

The sterile air which is injected into the culture medium is generally used in amounts such as to maintain in the medium an oxygen concentration higher than 20% of the saturation value.

The production of the AB-021 antibiotics, during the fermentation, can be monitored by means of antibiotic activity tests on broth samples, using microbial and fungal species sensitive to said antibiotics, or by HPLC analysis.

The fermentation is carried out for a time sufficient for obtaining a substantial antibiotic activity; 24 to 150 hours are generally sufficient for said purpose.

### Separation and purification of the Antibiotics

After the cultivation under the above fermentation conditions the AB-021 antibiotics and the main components thereof,i.e.AB-021 a and AB-021 b, can be separated from the culture broth and subsequently purified by means of methods conventional in the art of fermentation.

Such methods include, e.g., extraction with solvents, precipitation with non-solvents, ultrafiltration, column chromatography, silica-gel chromatography, cellulose chromatography, reverse-phase chromatography, chromatography on non-ionic, macroporous resins, and the like.

The antibiotics produced during the fermentation can be found in the culture broth and/or in the mycelium mass.

A preferred method for recovering AB-021 antibiotics consists in filtering off the mycelium mass from the culture broth, subjecting the mycelium thus separated to an extraction with acetone or methanol and concentrating the extract under vacuum until complete removal of the solvent. Thereby an aqueous suspension is obtained.

The thus obtained culture broth containing the AB-021 antibiotics is filtered through fibreglass filters and is then percolated on a column of a non-ionic polystyrene resin, such as, e.g., XAD-2 resin (Rohm & Haas Co.), which adsorbs the AB-021 antibiotics.

The resin is then washed with two volumes, based on its bed, of water, and thereafter is eluted with three volumes, also based on its bed, of an 8:2 (V/V) mixture of acetone:water.

The fractions containing AB-021 antibiotics, identified by means of biological activity tests on Botrytis, are combined and are then concentrated under vacuum until the disappearance of solvent, and the resulting aqueous suspension is combined with the one previously obtained from the mycelium to yield a crude product containing AB-021 antibiotics.

The two pure components AB-021 a and AB-021 b are then isolated from the crude product by means of reverse phase chromatography, using a column packed with silica (MATREXO silica C18 sold by Amicon Europe, Lausanne, Switzerland) with an eluent system formed by an eluent "A" consisting of water containing 10 mM monohydrogen ammonium phosphate and by an eluent "B" consisting of methanol, using a linear gradient of from 30% up to 70% of eluent "B" in eluent "A".

The fractions which contain antibiotic AB-021 a in pure form and antibiotic AB-021 b in pure form are separately concentrated under vacuum until complete removal of methanol. By cooling the remaining aqueous solutions down to 2 _{°} C, the precipitation of antibiotic AB-021 a and antibiotic AB-021 b respectively, is effected. Thereafter said antibiotics are centrifuged off. AB-021 a and AB-021 b, separated from their respective solutions as described above, are suspended in water and centrifuged again and, after removing the supernatant solutions and drying under vacuum at 40 _{°} C for 2 hours, pure antibiotic AB-021 a and pure antibiotic AB-021 b, respectively are obtained.

### Biological activity

The AB-021 antibiotics and the components thereof, i.e., AB-021 a and AB-021 b, are endowed with antifungal and antibacterial activity.

Their antifungal activity is particularly high against phytopathogenic fungi which infest agrarian cultivations of cereals, fruit trees and horticultural cultivations, as well as against pathogenic fungi in man.

The antifungal activities, both in vitro and in vivo, of the AB-021 antibiotics were determined by means of the methods described in the following:

### Tests for "in vitro" antifungal and bactericidal activity

The antimicrobial activity of AB-021 antibiotics was determined by the usual methods, by adding increasing concentrations of the antibiotic to an agarized culture medium capable of supporting the growth of the sensitive microbial species.

For phytopathogenic fungi, the minimum concentration of AB-021 antibiotics which under these conditions causes a reduction in mycelial growth of at least 50% as referred to the control (EDso) was furthermore determined. For yeast (Candida albicans) and bacteria (Sarcina lutea), the minimum concentration which completely inhibits growth (MIC) was determined.

In Tables D and E the data of biological activity which were obtained in vitro by means of the just described methods are reported.

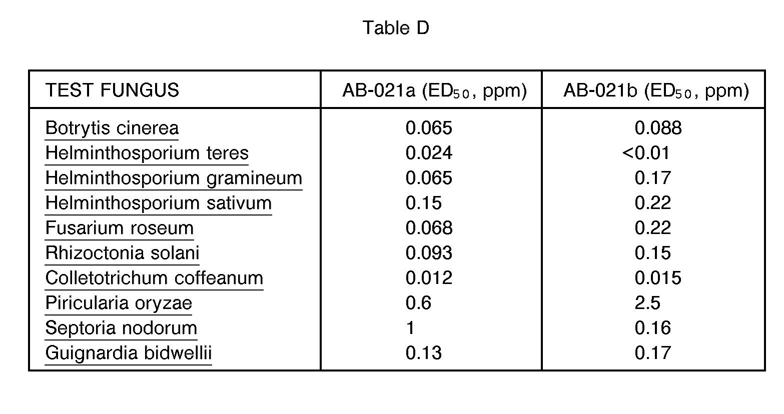

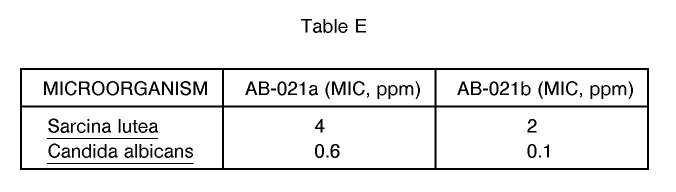

### Fungicidal activity "in vivo"

The fungicidal activity in vivo was measured by using the following method. The AB-021 antibiotics in water-acetone solution (20% acetone V/V) were sprayed onto the lower sides of leaves of plants grown in pots inside a conditioned room.

One day later, an inoculum of the tested fungus was sprayed on the upper sides of said plant leaves and the latter were then kept under incubation conditions inside a conditioned room for about eight days.

At the end of said time, the seriousness of the infection was evaluated by means of an evaluation scale ranging from 100 (= healthy plant) down to 0 (= completely infected plant).

The data concerning the preventive activity in vivo as obtained by means of the just described method is reported in Table E.

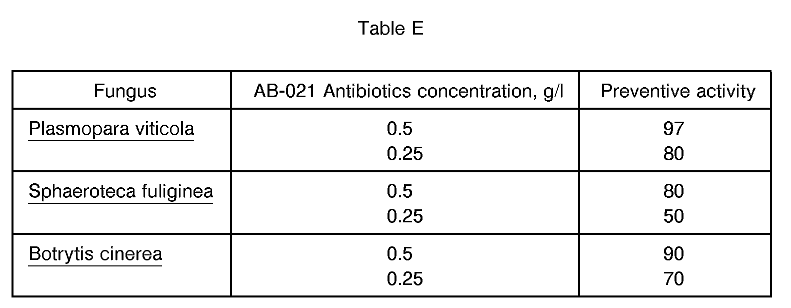

Strictly analogous results of antifungal activity were obtained when the individual antibiotics AB-021 a and AB-021 b were employed separately.

For their practical application, both in agriculture and in other sectors uf use, the antibiotics according to the present invention are preferably employed in the form of suitable formulations.

These formulations contain, besides the antibiotic according to the present invention as their active principle, inert solid carriers (e.g., kaolin, silica, talc, attapulgite, diatomaceous earth, etc.) or inert liquid carriers (e.g., organic solvents, vegetable or mineral oils, water and mixtures thereof), and optionally other additives which are normally used in the art of formulations, such as surfactants, suspending agents, dispersants and wetting agents.

In case of special application requirements, or in order to expand the range of action of the formulations, other active ingredients, such as, e.g., other insecticides, herbicides and fungicides can be added to the above compositions.

The doses to be applied vary as a function of different factors, such as the type and the degree of infestation, the type of composition used and climatic and environmental factors.

For practical use in agriculture, doses of AB-021 antibiotics usually ranging from 10 to 500 g/ha yield satisfactory results.

The following examples serve to illustrate the present invention without limiting it.

### Example 1

A culture of Streptomyces s p. NCIB 40068 grown on a plate of medium "A" (PM8) described above, thickened with agar, was suspended in 5 ml of sterile distilled water. Said suspension was used to inoculate an Erlenmeyer flask containing 100 ml of medium "A" (PM8) which subsequently was kept stirred at 180 rpm, at 28 _{°} C for 55 hours. Said culture was used to inoculate an Erlenmeyer flask containing 1000 ml of medium "A" (PM8) which subsequently was kept stirred at 150 rpm, at 28 ° C for 40 hours. With the resulting culture a fermenter of 40 litres rated capacity, containing 28 litres of medium "B" (PGC), was then inoculated.

While the temperature was kept at 28 ° C and the concentration of dissolved oxygen was maintained above 20% of the saturation value, the fermentation was carried out for 74 hours.

### Separation of AB-021 Antibiotics

28 litres of fermentation broth obtained as described above were centrifuged and the mycelium thus separated was extracted with 7 litres of acetone.

The acetone extract was concentrated under vacuum until the solvent was completely removed and the residual aqueous solution containing the AB-021 antibiotics (about 1 litre) was combined with 400 ml of methanol and was chromatographed on a column (inner diameter 90 mm, length 360 mm) containing 1.0 kg of silica (MATREXO Silica C18) with a forecolumn (50 mm x 50 mm) packed with the same stationary phase, using an eluent system which was formed by an eluent "A" constituted by water containing 10 mM monohydrogen ammonium phosphate and an eluent "B" constituted by methanol, the gradient being as indicated in the following Table:

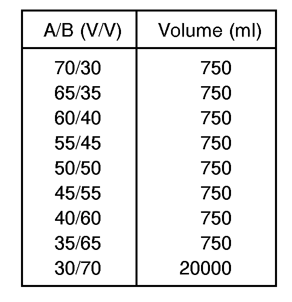

Pure AB-021 a antibiotic was contained in 2550 ml within the elution range of from 15000 to 17550 ml, while AB-021 b antibiotic was contained in 3000 ml within the elution range of from 21450 to 24450 ml.

The fractions thus collected were evaporated under vacuum until the methanol was completely removed, and were then left to stand for 24 hours at 4 ° C. From these solutions, AB-021 a and AB-021 b, respectively precipitated and were then isolated by centrifugation. Thereafter the centrifuge cakes were suspended again in water in order to remove any residual salt traces, and were then centrifuged again.

After drying, 198 mg of antibiotic AB-021 a, a light-yellow coloured powder, and 210 mg of antibiotic AB-021 b, a yellow-coloured powder, were obtained.

The physico-chemical characteristics of the products have been reported above.

### Example 2

A culture of Streptomyces s p. NCIB 40068 grown on a plate of agarized medium "A" (PM8) was suspended in 6 ml of sterile distilled water. Said suspension was used to inoculate 3 Erlenmeyer flasks, each containing 100 ml of medium "A" (PM8). The inoculated Erlenmeyer flasks were then kept stirred at 180 rpm, at 28 ° C for 48 hours. The culture thus obtained was used to inoculate 50 Erlenmeyer flasks, each containing 100 ml of medium "B" (PGC), with an inoculum of 5% of the volume. The Erlenmeyer flasks were kept stirred at 180 rpm, at 28 ° C for 72 hours. The resulting broth was then collected and separated from the mycelium by means of centrifugation at 6000 rpm for 20 minutes.

### Example 3

A culture of Streptomyces s p. NCIB 40068,grown on a plate of agarized medium "A" (PM8),was suspended in 5 ml of sterile distilled water. Said suspension was used to inoculate an Erlenmeyer flask containing 100 ml of medium "A" (PM8). The inoculated Erlenmeyer flask was then kept stirred at 180 rpm, at 28 _{°} C for 48 hours.

The culture thus obtained was used to inoculate 3 Erlenmeyer flasks, each containing 100 ml of medium "C" (SCM), with an inoculum of 5% of the volume. The Erlenmeyer flasks were kept stirred at 180 rpm, at 30 ° C for 24-72 hours. At the end of the fermentation, the resulting broth was separated from the mycelium by means of centrifugation which was carried out in the same way as in Example 2.

## Claims (Claims for the following Contracting State(s) : AT, BE, CH, DE, FR, GB, LI, NL, SE)

1. Antibiotic AB-021 a, a solid substance characterized by:
(a) the following approximate elemental analysis: 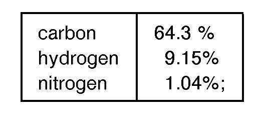
(b) molecular weight of about 1139;
(c) UV-absorbance maxima of: 0.193 at 232.8 nm; 2.064 at 318.4 nm; 2.44 at 332.6 nm; 2.113 at 349.6 nm, at a concentration of 0.025 mg/ml in acetonitrile/water 1:1 (V/V);
(d) IR-absorbance maxima at (cm-¹): 3365; 3015; 2923; 2851; 1700; 1634; 1594; 1541; 1431; 1380; 1329; 1262; 1128; 1095; 1056; 1004; 968; 919; 878; 843; 804; 754;
(e) main peaks in its ¹H-NMR spectrum at:
6TMS (ppm) : 7.18 (d, 1 H); 6.69 (dd, 1 H); 6.59 (dd, 1 H); 6.52 - 6.09 (m, 9H); 5.96 - 5.70 (m, 3H); 5.70 - 5.31 (m, 8H); 4.27 - 4.02 (m, 4H); 4.02 - 3.82 (m, 4H); 3.82 - 3.58 (m, 4H); 3.30 (t, 1 H); 2.82 (t, 2H); 2.73 - 2.45 (^{*}) (m, 3H); 2.41 - 1.98 (m, 13H); 1.91 (s, 3H); 1.79 - 1.12 (m, 24-25H); 1.00 (d, 3H); 0.95 (d, 3H); 0.89 (d, 3H); 0.85 (d, 3H);
(f) main peaks in its ¹³C-NMR spectrum at:
6TMS (ppm) : 212.1 (s); 169.9 (s); 138.8 (d); 138.5 (d); 137.0 (d); 136.9 (d); 136.7 (d); 136.1 (d); 135.9 (d); 135.9 (d); 134.0 (d); 133.3 (d); 133.0 (d); 132.9 (d); 132.5 (d); 132.4 (d); 132.0 (d); 131.5 (d); 131.0 (d); 131.0 (d); 129.3 (d); 128.9 (d); 128.0 (d); 126.7 (d); 126.4 (d); 125.3 (d); 75.3 (d); 72.3 (d); 71.2 (d); 70.6 (d); 69.7 (d);
((^{*}) Within this range, also the peak of DMSO-d6 appears); 68.9 (d); 68.4 (d); 68.0 (d); 67.1 (d); 67.1 (d); 65.7 (d); 64.2 (d); 64.2 (d); 52.1 (d); 41.5 (d); 39.9 (d); 49.2 (t); 46.2 (t); 46.0 (t); 45.2 (t); 45.2 (t); 44.9 (t); 43.9 (t); 41.2 (t); 40.8 (t); 40.7 (t); 39.2 (t); 38.8 (t); 34.1 (t); 32.8 (t); 32.1 (t); 29.3 (t); 24.1 (t); 22.8 (q); 18.4 (q); 13.2 (q); 10.5 (q); 9.1 (q).
(g) Rₜ values obtained by thin-layer chromatography (TLC) on plates of the type 60F 254 (Merck-Schuchardt):
0.47-0.57 in ethanol : dioxane : aqueous solution of ammonia (30%) : water (8:1:1:1);
0.0 in methylene chloride:methanol (17:3);
R_{f} values obtained by reverse-phase chromatography on plates of the type RP-18F 254 (Merck-Schuchardt):
0.29 in methanol : acetonitrile : 25 mM monohydrogen sodium phosphate in water (4:4:2);
0.39 in methanol : acetonitrile : 25 mM dihydrogen potassium phosphate + 7 mM tetramethylammonium chloride in water (4:4:2);
0.21 in methanol : 10 mM aqueous solution of monohydrogen ammonium phosphate adjusted to pH 7.5 with phosphoric acid (8:2);
0.25 in methanol : acetonitrile : 10 mM aqueous solution of monohydrogen ammonium phosphate adjusted to pH 7.5 with phosphoric acid (4:4:2).
(h) a retention time (Rₜ) of 8 minutes in reverse-phase HPLC on a Hibar Li-ChroCARTO Li-Chrosorb RP-18 column, (forecolumn: Guard Pak@ RCSS C 18) when eluted with a methanol : acetonitrile : 10 mM aqueous solution of monohydrogen ammonium phosphate (1:1:1) mixture at a flow rate of 0.8 ml/minute and 40 ° C;
(i) a good solubility in dimethylsulfoxide and in ethanol/water (1:1 V/V) or methanol/water (1:1 V/V) mixtures, scarce solubility in water and fairly good solubility in ethanol and methanol.

2. Antibiotic AB-021 b, a solid substance characterized by:
(a) the following approximate elemental analysis: 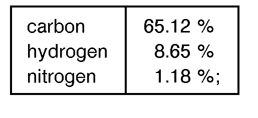
(b) molecular weight of about 1165;
(c) UV-absorbance maxima of: 0.90 at 369.0 nm; 2.01 at 350.0 nm; 2.21 at 332.6 nm; 1.49 at 318.0 nm;at a concentration of 0.02 mg/ml in acetonitrile/water 1:1 (V/V);
(d) IR-absorbance maxima at (cm-¹): 3902, 3853, 3747, 3375, 3013, 2921, 2853, 2757, 1895, 1699, 1669, 1645, 1576, 1540, 1430, 1378, 1324, 1261, 1160, 1094, 1059, 1004, 969, 919, 878, 843, 803, 779, 697;
(e) main peaks in its ¹H-NMR spectrum at:
δTMS (ppm) : 7.21 (d, 1 H); 6.75 (dd, 1 H); 6.63 (dd, 1 H); 6.57 - 6.07 (m, 11 H); 5.93 - 5.70 (m, 3H); 5.70 - 5.33 (m, 8H); 4.25 - 4.20 (m, 4H); 4.02 - 3.83 (m, 4H); 3.83 - 3.59 (m, 4H); 3.29 (t, 1 H); 2.82 (t, 2H); 2.72 - 2.44 (^{*}) (m, 6-7H); 2.41 - 2.00 (m, 14H); 1.92 (s, 3H); 1.80 - 1.14 (m, 21 H); 1.00 (d, 3H); 0.95 (d, 3H); 0.84 (d, 3H).
(f) main peaks in its ¹³C-NMR spectrum at:
δTMS (ppm) : 212.0 (s); 169.2 (s); 139.1 (d); 138.1 (d); 137.1 (d); 136.7 (d); 136.2 (d); 135.8 (d); 135.8 (d); 135.1 (d); 133.8 (d); 133.1 (d); 132.8 (d); 132.8 (d); 132.6 (d); 132.3 (d); 132.2 (d); 132.1 (d); 131.8 (d); 131.2 (d); 130.9 (d); 130.8 (d); 129.2 (d); 128.7 (d); 127.9 (d); 126.4 (d); 126.2 (d); 125.2 (d); 75.4 (d); 72.6 (d); 71.0 (d); 70.6 (d); 69.6 (d); 68.9 (d); 68.4 (d); 68.0 (d); 67.3 (d); 67.2 (d); 65.6 (d); 64.4 (d); 64.4 (d); 51.9 (d); 41.3 (d) 39.6 (d); 49.2 (t); 45.8 (t); 45.7 (t); 45.0 (t); 44.9 (t); 44.7 (t); 43.6 (t); 41.1 (t); 40.7 (t); 40.5 (t); 39.0 (t); 38.7 (t); 33.9 (t); 32.5 (t); 31.9 (t); 29.0 (t); 23.9 (t); 18.1 (q); 12.8 (q); 10.4 (q); 8.7 (q);
(g) R_{f} values obtained by thin-layer chromatography (TLC) on plates of the type 60F 254 (Merck-Schuchardt):
((^{*}) Within this range also the peak of DMSO-d6 appears); DMSO-d6 appears);
0.54 in ethanol : dioxane : aqueous solution of ammonia (30%) : water (8:1:1:1); 0.00 in methylene chloride:methanol (17:3);
Rₜ values obtained by reverse-phase chromatography on plates of the type RP-18F 254 (Merck-Schuchardt):
0.27 in methanol : acetonitrile : 25 mM monohydrogen sodium phosphate in water (4:4:2)
0.35 in methanol : acetonitrile : 25 mM dihydrogen potassium phosphate in water + 7 mM tetramethylammonium chloride in water (4:4:2)
0.12 in methanol : 10 mM aqueous solution of monohydrogen ammonium phosphate adjusted to pH 7.5 with phosphoric acid (8:2)
0.24 in methanol : acetonitrile : 10 mM aqueous solution of monohydrogen ammonium phosphate adjusted to pH 7.5 with phosphoric acid (4:4:2);
(h) a retention time (Rₜ) of 11.8 minutes in reverse-phase HPLC on a Hibar Li-ChroCARTO Li-Chrosorb@ RP18 column, (forecolumn: Guard Pak@ RCSS C18) when eluted with a methanol : acetonitrile : 10 mM aqueous solution of monohydrogen ammonium phosphate (1:1:1) mixture at a flow rate of 0.8 ml/minute and at 40 _{°} C.

3. AB-021 antibiotics, obtainable by means of the controlled cultivation under aerobic conditions of Streptomyces s p. NCIB 40068 or of an equivalent mutant thereof in an aqueous nutrient cultivation medium containing sources of carbon, nitrogen and inorganic salts, said antibiotics being substantially composed of antibiotic AB-021 a and antibiotic AB-021 b as defined in claims 1 and 2.

4. Process for the preparation of AB-021 antibiotics, comprising the cultivation of Streptomyces s p. NCIB 40068 or of a corresponding mutant thereof under conditions of controlled aerobic fermentation in an aqueous nutrient medium containing assimilatable sources of carbon and nitrogen as well as inorganic salts until a substantial antibiotic activity is obtained, the subsequent recovery of said antibiotics by means of per se known methods, and, optionally, the separation of the main components, designated AB-021 a and AB-021 b, as defined in claims 1 and 2.

5. Process according to claim 4, in which the fermentation is carried out at a temperature of from 20 _{°} C to 35_{°}C.

6. Process according to any one of claims 4 and 5, in which the fermentation is carried out at a pH within the range of from 5 to 9.

7. Process according to any one of claims 4 to 6, in which the AB-021 antibiotics are isolated from the fermentation broth by means of filtration and subsequent use of chromatographic techniques.

8. Process according to any one of claims 4 to 7, in which AB-021 a and AB-021 b are isolated by reverse-phase chromatography on a silica-gel column using a linear elution gradient of from 30% to 70% of methanol in the corresponding mixture with water containing 10 mM/I of monohydrogen ammonium phosphate.

9. Microorganism Streptomyces s p. NCIB 40068.

10. The biologically pure culture of Streptomyces s p. NCIB 40068 or a equivalent mutant thereof, capable of producing AB-021 antibiotics in isolatable amounts by means of the controlled aerobic fermentation in an aqueous nutrient medium comprising assimilatable sources of carbon and nitrogen as well as inorganic salts.

11. Fungicidal and/or bactericidal compositions containing as active ingredient a compound selected from AB-021 antibiotics, AB-021 a and AB-021 b, together with inert solid or liquid carriers and, optionally, other additives.

## Claims (Claims for the following Contracting State(s) : ES)

1. Process for the preparation of AB-021 a antibiotic, characterized in that it comprises the cultivation of Streptomyces s p. NCIB 40068 or of a corresponding mutant thereof under conditions of controlled aerobic fermentation in an aqueous nutrient medium containing assimilatable sources of carbon and nitrogen as well as inorganic salts until a substantial antibiotic activity is obtained, the subsequent recovery of said antibiotic by means of per se known methods, and the separation of AB-021a a antibiotic by reverse-phase chromatography on a silica-gel column using a linear elution gradient of from 30% to 70% of methanol in the corresponding mixture with water containing 10 mM/I of monohydrogen ammonium phosphate, the recovered solid substance having the following properties:
(a) the following approximate elemental analysis: 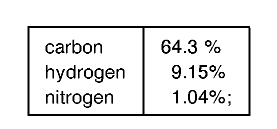
(b) molecular weight of about 1139;
(c) UV-absorbance maxima of: 0.193 at 232.8 nm; 2.064 at 318.4 nm; 2.44 at 332.6 nm; 2.113 at 349.6 nm, at a concentration of 0.025 mg/ml in acetonitrile/water 1:1 (V/V);
(d) IR-absorbance maxima at (cm-¹): 3365; 3015; 2923; 2851; 1700; 1634; 1594; 1541; 1431; 1380; 1329; 1262; 1128; 1095; 1056; 1004; 968; 919; 878; 843; 804; 754;
(e) main peaks in its ¹H-NMR spectrum at:
δTMS (ppm) : 7.18 (d, 1 H); 6.69 (dd, 1 H); 6.59 (dd, 1 H); 6.52 - 6.09 (m, 9H); 5.96 - 5.70 (m, 3H); 5.70 - 5.31 (m, 8H); 4.27 - 4.02 (m, 4H); 4.02 - 3.82 (m, 4H); 3.82 - 3.58 (m, 4H); 3.30 (t, 1 H); 2.82 (t, 2H); 2.73 - 2.45 (^{*}) (m, 3H); 2.41 - 1.98 (m, 13H); 1.91 (s, 3H); 1.79 - 1.12 (m, 24-25H); 1.00 (d, 3H); 0.95 (d, 3H); 0.89 (d, 3H); 0.85 (d, 3H);
(f) main peaks in its ¹³C-NMR spectrum at:
δTMS (ppm) : 212.1 (s); 169.9 (s); 138.8 (d); 138.5 (d); 137.0 (d); 136.9 (d); 136.7 (d); 136.1 (d); 135.9 (d); 135.9 (d); 134.0 (d); 133.3 (d); 133.0 (d); 132.9 (d); 132.5 (d); 132.4 (d); 132.0 (d); 131.5 (d); 131.0 (d); 131.0 (d); 129.3 (d); 128.9 (d); 128.0 (d); 126.7 (d); 126.4 (d); 125.3 (d); 75.3 (d); 72.3 (d); 71.2 (d); 70.6 (d); 69.7 (d); 68.9 (d); 68.4 (d); 68.0 (d); 67.1 (d); 67.1 (d); 65.7 (d); 64.2 (d); 64.2 (d); 52.1 (d); 41.5 (d); 39.9 (d); 49.2 (t); 46.2 (t); 46.0 (t); 45.2 (t); 45.2 (t); 44.9 (t); 43.9 (t); 41.2 (t); 40.8 (t); 40.7 (t); 39.2 (t); 38.8 (t); 34.1 (t); 32.8 (t); 32.1 (t); 29.3 (t); 24.1 (t); 22.8 (q); 18.4 (q); 13.2 (q); 10.5 (q); 9.1 (q).
(g) R_{f} values obtained by thin-layer chromatography (TLC) on plates of the type 60F 254 (Merck-Schuchardt):
0.47-0.57 in ethanol : dioxane : aqueous solution of ammonia (30%) : water (8:1:1:1);
0.0 in methylene chloride:methanol (17:3);
R_{f} values obtained by reverse-phase chromatography on plates of the type RP-18F 254 (Merck-Schuchardt):
0.29 in methanol : acetonitrile : 25 mM monohydrogen sodium phosphate in water (4:4:2);
0.39 in methanol : acetonitrile : 25 mM dihydrogen potassium phosphate + 7 mM tetramethylammonium chloride in water (4:4:2);
0.21 in methanol : 10 mM aqueous solution of monohydrogen ammonium phosphate adjusted to pH 7.5 with phosphoric acid (8:2);
0.25 in methanol : acetonitrile : 10 mM aqueous solution of monohydrogen ammonium phosphate adjusted to pH 7.5 with phosphoric acid (4:4:2);
(h) a retention time (Rₜ) of 8 minutes in reverse-phase HPLC on a Hibar Li-ChroCARTO Li-Chrosorb RP-18 column, (forecolumn: Guard Pak@ RCSS C 18) when eluted with a methanol : acetonitrile : 10 mM aqueous solution of monohydrogen ammonium phosphate (1:1:1) mixture at a flow rate of 0.8 ml/minute and 40 ° C;
(i) a good solubility in dimethylsulfoxide and in ethanol/water (1:1 V/V) or methanol/water (1:1 V/V) mixtures, scarce solubility in water and fairly good solubility in ethanol and methanol.
((^{*}) Within this range, also the peak of DMSO-d6 appears);

2. Process for the preparation of AB-021 b antibiotic, characterized in that it comprises
the cultivation of Streptomyces s p. NCIB 40068 or of a corresponding mutant thereof under conditions of controlled aerobic fermentation in an aqueous nutrient medium containing assimilatable sources of carbon and nitrogen as well as inorganic salts until a substantial antibiotic activity is obtained,
the subsequent recovery of said antibiotic by means of per se known methods, and
the separation of AB-021 b antibiotic by reverse-phase chromatography on a silica-gel column using a linear elution gradient of from 30% to 70% of methanol in the corresponding mixture with water containing 10 mM/I of monohydrogen ammonium phosphate,
the recovered solid substance having the following properties:
(a) the following approximate elemental analysis: 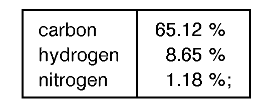
(b) molecular weight of about 1165;
(c) UV-absorbance maxima of: 0.90 at 369.0 nm; 2.01 at 350.0 nm; 2.21 at 332.6 nm; 1.49 at 318.0 nm;at a concentration of 0.02 mg/ml in acetonitrile/water 1:1 (V/V);
(d) IR-absorbance maxima at (cm-¹):
3902, 3853, 3747, 3375, 3013, 2921, 2853, 2757, 1895, 1699, 1669, 1645, 1576, 1540, 1430, 1378, 1324, 1261, 1160, 1094, 1059, 1004, 969, 919, 878, 843, 803, 779, 697;
(e) main peaks in its ¹H-NMR spectrum at:
6TMS (ppm) : 7.21 (d, 1 H); 6.75 (dd, 1 H); 6.63 (dd, 1 H); 6.57 - 6.07 (m, 11 H); 5.93 - 5.70 (m, 3H); 5.70 - 5.33 (m, 8H); 4.25 - 4.20 (m, 4H); 4.02 - 3.83 (m, 4H); 3.83 - 3.59 (m, 4H); 3.29 (t, 1 H); 2.82 (t, 2H); 2.72 - 2.44 (^{*}) (m, 6-7H); 2.41 - 2.00 (m, 14H); 1.92 (s, 3H); 1.80 - 1.14 (m, 21 H); 1.00 (d, 3H); 0.95 (d, 3H); 0.84 (d, 3H).
(f) main peaks in its ¹³C-NMR spectrum at:
δTMS (ppm) : 212.0 (s); 169.2 (s); 139.1 (d); 138.1 (d); 137.1 (d); 136.7 (d); 136.2 (d); 135.8 (d); 135.8 (d); 135.1 (d); 133.8 (d); 133.1 (d); 132.8 (d); 132.8 (d); 132.6 (d); 132.3 (d); 132.2 (d); 132.1 (d); 131.8 (d); 131.2 (d); 130.9 (d); 130.8 (d); 129.2 (d); 128.7 (d); 127.9 (d); 126.4 (d); 126.2 (d); 125.2 (d); 75.4 (d); 72.6 (d); 71.0 (d); 70.6 (d); 69.6 (d); 68.9 (d); 68.4 (d); 68.0 (d); 67.3 (d); 67.2 (d); 65.6 (d); 64.4 (d); 64.4 (d); 51.9 (d); 41.3 (d); 39.6 (d); 49.2 (t); 45.8 (t); 45.7 (t); 45.0 (t); 44.9 (t); 44.7 (t); 43.6 (t); 41.1 (t); 40.7 (t); 40.5 (t); 39.0 (t); 38.7 (t); 33.9 (t); 32.5 (t); 31.9 (t); 29.0 (t); 23.9 (t); 18.1 (q); 12.8 (q); 10.4 (q); 8.7 (q);
(g) Rₜ values obtained by thin-layer chromatography (TLC) on plates of the type 60F 254 (Merck-Schuchardt):
0.54 in ethanol : dioxane : aqueous solution of ammonia (30%) : water (8:1:1:1);
0.00 in methylene chloride:methanol (17:3);
Rₜ values obtained by reverse-phase chromatography on plates of the type RP-18F 254 (Merck-Schuchardt):
0.27 in methanol : acetonitrile : 25 mM monohydrogen sodium phosphate in water (4:4:2)
0.35 in methanol : acetonitrile : 25 mM dihydrogen potassium phosphate in water + 7 mM tetramethylammonium chloride in water (4:4:2)
0.12 in methanol : 10 mM aqueous solution of monohydrogen ammonium phosphate adjusted to pH 7.5 with phosphoric acid (8:2)
0.24 in methanol : acetonitrile : 10 mM aqueous solution of monohydrogen ammonium phosphate adjusted to pH 7.5 with phosphoric acid (4:4:2);
(h) a retention time (Rₜ) of 11.8 minutes in reverse-phase HPLC on a Hibar Li-ChroCARTO Li-Chrosorb@ RP18 column, (forecolumn: Guard Pak@ RCSS C18) then eluted with a methanol : acetonitrile : 10 mM aqueous solution of monohydrogen ammonium phosphate (1:1:1) mixture at a flow rate of 0.8 ml/minute and at 40 ° C.
((^{*}) Within this range also the peak of DMSO-d6 appears);

3. Process for the preparation of AB-021 antibiotics, that antibiotics being substantially composed of antibiotic AB-021 a and antibiotic AB-021 b as defined in claims 1 and 2, characterized in that it comprises the cultivation of Streptomyces s p. NCIB 40068 or of a corresponding mutant thereof under conditions of controlled aerobic fermentation in an aqueous nutrient medium containing assimilatable sources of carbon and nitrogen as well as inorganic salts until a substantial antibiotic activity is obtained, and the subsequent recovery of said antibiotics by means of per se known methods.

4. Process according to any one of claims 1 to 3, characterized in that the fermentation is carried out at a temperature of from 20 ° C to 35 ° C.

5. Process according to any one of claims 1 to 4, characterized in that the fermentation is carried out at a pH within the range of from 5 to 9.

6. Process according to any one of claims 1 to 5, characterized in that the AB-021 antibiotics are isolated from the fermentation broth by means of filtration and subsequent use of chromatographic techniques.

## Patentansprüche (Patentansprüche für folgende(n) Vertragsstaat(en) : AT, BE, CH, DE, FR, GB, LI, NL, SE)

1. Das Antibiotikum AB-021 a, welches als feste Substanz vorliegt, gekennzeichnet durch:
(a) die folgende angenäherte Elementaranalyse: 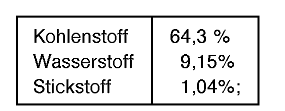
(b) ein Molekulargewicht von etwa 1139;
(c) UV-Absorptionsmaxima von: 0,193 bei 232,8 nm; 2,064 bei 318,4 nm; 2,44 bei 332,6 nm; 2,113 bei 349,6 nm; bei einer Konzentration von 0,025 mg/ml in Acetonitril/Wasser 1:1 (Vol./Vol.);
(d) IR-Absorptionsmaxima bei (cm⁻¹): 3365; 3015; 2923; 2851; 1700; 1634; 1594; 1541; 1431; 1380; 1329; 1262; 1128; 1095; 1056; 1004; 968; 919; 878; 843; 804; 754;
(e) Hauptpeaks in seinem ¹H-NMR Spektrum bei: δTMS (ppm): 7,18 (d, 1 H); 6,69 (dd, 1 H); 6,59 (dd, 1 H); 6,52 - 6,09 (m, 9H); 5,96 - 5,70 (m, 3H); 5,70
- 5,31 (m, 8H); 4,27 - 4,02 (m, 4H); 4,02 - 3,82 (m, 4 H); 3,82 - 3,58 (m, 4H); 3,30 (t, 1 H); 2,82 (t, 2H); 2,73 - 2,45 (^{*}) (m, 3H); 2,41 - 1,98 (m, 13H); 1,91 (s, 3H); 1,79 - 1,12 (m, 24-25H); 1,00 (d, 3H); 0,95 (d, 3H); 0,89 (d, 3H); 0,85 (d, 3H);
(f) Hauptpeaks in seinem ¹³C-NMR Spektrum bei:
δTMS (ppm): 212,1 (s); 169,9 (s); 138,8 (d); 138,5 (d); 137,0 (d); 136,9 (d); 136,7 (d); 136,1 (d); 135,9 (d); 135,9 (d); 134,0 (d); 133,3 (d); 133,0 (d); 132,9 (d); 132,5 (d); 132,4 (d); 132,0 (d); 131,5 (d); 131,0 (d); 131,0 (d); 129,3 (d); 128,9 (d); 128,0 (d); 126,7 (d); 126,4 (d); 125,3 (d); 75,3 (d); 72,3 (d); 71,2 (d); 70,6 (d); 69,7 (d); 68,9 (d); 68,4 (d); 68,0 (d); 67,1 (d); 67,1 (d); 65,7 (d); 64,2 (d); 64,2 (d); 52,1 (d); 41,5 (d); 39,9 (d); 49,2 (t); 46,2 (t); 46,0 (t); 45,2 (t); 45,2 (t); 44,9 (t); 43,9 (t); 41,2 (t); 40,8 (t); 40,7 (t); 39,2 (t); 38,8 (t); 34,1 (t); 32,8 (t); 32,1 (t); 29,3 (t); 24,1 (t); 22,8 (q); 18,4 (q); 13,2 (q); 10,5 (q); 9,1 (q);
(g) mittels Dünnschicht-Chromatographie (TLC) auf Platten des Typs 60F 254 (Merck-Schuchardt) erhaltene R_{f}-Werte von:
0,47-0,57 in Ethanol : Dioxan : wäßrige Ammoniaklösung (30%): Wasser (8:1:1:1);
0,0 in Methylenchlorid : Methanol (17:3);
mittels Umkehrphasen-Chromatographie auf Platten des Typs RP-18F 254 (Merck-Schuchardt) erhaltene R_{f}-Werte von:
0,29 in Methanol : Acetonitril : 25 mM Natriummonohydrogenphosphat in Wasser (4:4:2);
0,39 in Methanol : Acetonitril : 25 mM Kaliumdihydrogenphosphat + 7 mM Tetramethylammoniumchlorid in Wasser (4:4:2);
((^{*}) der Peak des DMSO-d6 erscheint ebenfalls innerhalb dieses Bereichs);
0,21 in Methanol : 10 mM wäßrige, mittels Phosphorsäure auf einen pH-Wert von 7,5 eingestellte Lösung von Ammoniummonohydrogenphosphat (8:2);
0,25 in Methanol : Acetonitril : 10 mM wäßrige, mittels Phosphorsäure auf einen pH-Wert von 7,5 eingestellte Lösung von Ammoniummonohydrogenphosphat (4:4:2);
(h) eine Retentionszeit (Rₜ) von 8 Minuten bei der Umkehrphasen-HPLC auf einer Hibar Li-ChroCART® Li-Chrosorb RP-18 Säule (Vorsäule: Guard Pak@ RCSS C 18) unter Verwendung einer Elutionsmischung aus Methanol : Acetonitril : 10 mM wäßrige Lösung von Ammoniummonohydrogenphosphat(1:1:1) bei einer Strömungsgeschwindigkeit von 0,8 ml/min sowie 40 ° C;
(i) eine gute Löslichkeit in Dimethylsulfoxid und in Mischungen aus Ethanol/Wasser (1:1 Vol./Vol.) oder Methanol/Wasser (1:1 Vol./Vol.), sowie schlechte Löslichkeit in Wasser und recht gute Löslichkeit in Ethanol und Methanol.

2. Das Antibiotikum AB-021 b, welches als feste Substanz vorliegt, gekennzeichnet durch:
(a) die folgende angenäherte Elementaranalyse: 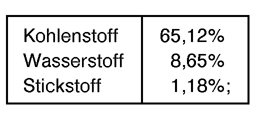
(b) ein Molekulargewicht von etwa 1165;
(c) UV-Absorptionsmaxima von:
0,90 bei 369,0 nm; 2,01 bei 350,0 nm; 2,21 bei 332,6 nm; 1,49 bei 318,0 nm; bei einer Konzentration von 0,02 mg/ml in Acetonitril/Wasser 1:1 (Vol./Vol.);
(d) IR-Absorptionsmaxima bei (cm⁻¹):
3902, 3853, 3747, 3375, 3013, 2921, 2853, 2757, 1895, 1699, 1669, 1645, 1576, 1540, 1430, 1378, 1324, 1261, 1160, 1094, 1059, 1004, 969, 919, 878, 843, 803, 779, 697;
(e) Hauptpeaks in seinem ¹H-NMR Spektrum bei:
δTMS (ppm): 7,21 (d, 1 H); 6,75 (dd, 1 H); 6,63 (dd, 1 H); 6,57 - 6,07 (m, 11 H); 5,93 - 5,70 (m, 3H); 5,70 - 5,33 (m, 8H); 4,25 - 4,20 (m, 4H); 4,02 - 3,83 (m, 4 H); 3,83 - 3,59 (m, 4H); 3,29 (t, 1 H); 2,82 (t, 2H); 2,72 - 2,44 (^{*}) (m, 6-7H); 2,41 -2,00 (m, 14H); 1,92 (s, 3H); 1,80 - 1,14 (m, 21 H); 1,00 (d, 3H); 0,95 (d, 3H); 0,84 (d, 3H);
(f) Hauptpeaks in seinem ¹³C-NMR Spektrum bei:
δTMS (ppm): 212,0 (s); 169,2 (s); 139,1 (d); 138,1 (d); 137,1 (d); 136,7 (d); 136,2 (d); 135,8 (d); 135,8
(d); 135,1 (d); 133,8 (d); 133,1 (d); 132,8 (d); 132,8 (d); 132,6 (d); 132,3 (d); 132,2 (d); 132,1 (d); 131,8 (d); 131,2 (d); 130,9 (d); 130,8 (d); 129,2 (d); 128,7 (d); 127,9 (d); 126,4 (d); 126,2 (d); 125,2 (d); 75,4 (d); 72,6 (d); 71,0 (d); 70,6 (d); 69,6 (d); 68,9 (d); 68,4 (d); 68,0 (d); 67,3 (d); 67,2 (d); 65,6 (d); 64,4 (d); 64,4 (d); 51,9 (d); 41,3 (d); 39,6 (d); 49,2 (t); 45,8 (t); 45,7 (t); 45,0 (t); 44,9 (t); 44,7 (t); 43,6 (t); 41,1 (t); 40,7 (t); 40,5 (t); 39,0 (t); 38,7 (t); 33,9 (t); 32,5 (t); 31,9 (t); 29,0 (t); 23,9 (t); 18,1 (q); 12,8 (q); 10,4 (q); 8,7 (q);
(g) mittels Dünnschicht-Chromatographie (TLC) auf Platten des Typs 60F 254 (Merck-Schuchardt) erhaltene R_{f}-Werte von:
0,54 in Ethanol : Dioxan : wäßrige Ammoniaklösung (30%) : Wasser (8:1:1:1);
0,00 in Methylenchlorid : Methanol (17:3);
mittels Umkehrphasen-Chromatographie auf Platten des Typs RP-18F 254 (Merck-Schuchardt) erhaltene R_{f}-Werte von:
0,27 in Methanol : Acetonitril : 25 mM Natriummonohydrogenphosphat in Wasser (4:4:2);
0,35 in Methanol : Acetonitril : 25 mM Kaliumdihydrogenphosphat + 7 mM Tetramethylammoniumchlorid in Wasser (4:4:2);
0,12 in Methanol : 10 mM wäßrige, mittels Phosphorsäure auf einen pH-Wert von 7,5 eingestellte Lösung von Ammoniummonohydrogenphosphat (8:2);
0,24 in Methanol : Acetonitril : 10 mM wäßrige, mittels Phosphorsäure auf einen pH-Wert von 7,5 eingestellte Lösung von Ammoniummonohydrogenphosphat (4:4:2);
(h) eine Retentionszeit (Rₜ) von 11,8 Minuten bei der Umkehrphasen-HPLC auf einer Hibar Li-ChroCART® Li-Chrosorb® RP-18 Säule (Vorsäule: Guard Pak@ RCSS C18) unter Verwendung einer Elutionsmischung aus Methanol : Acetonitril : 10 mM wäßrige Lösung von Ammoniummonohydro-
((^{*}) der Peak des DMSO-d6 erscheint ebenfalls innerhalb dieses Bereiches);
genphosphat(1:1:1) bei einer Strömungsgeschwindigkeit von 0,8 ml/min sowie 40 ° C.

3. AB-021-Antibiotika, erhältlich mittels kontrollierter Kultivierung unter aeroben Bedingungen von Streptomyces s p. NCIB 40068 oder einer gleichwertigen Mutante davon in einem wäßrigen Kultivierungs-Nährmedium, welches Kohlenstoff- und Stickstoff-Quellen sowie anorganische Salze enthält, wobei diese Antibiotika im wesentlichen das Antibiotikum AB-021 a und das Antibiotikum AB-021 b gemäß der Definitionen in den Ansprüchen 1 und 2 beinhalten.

4. Verfahren zur Herstellung der AB-021-Antibiotika, umfassend eine Kultivierung unter kontrollierten aeroben Fermentationsbedingungen von Streptomyces s p. NCIB 40068 oder einer entsprechenden Mutante davon in einem wäßrigen Nährmedium, welches assimilierbare Kohlenstoff- und Stickstoff-Quellen sowie anorganische Salze enthält, bis eine beträchtliche antibiotische Aktivität erreicht ist, eine anschließende Gewinnung der besagten Antibiotika mittels an sich bekannter Verfahren und gegebenenfalls eine Trennung der Hauptkomponenten, welche gemäß der Definitionen in den Ansprüchen 1 und 2 als AB-021 a und AB-021 b bezeichnet werden.

5. Verfahren gemäß Anspruch 4, in dem die Fermentation bei einer Temperatur von 20 ° C bis 35 ° C durchgeführt wird.

6. Verfahren gemäß irgendeinem der Ansprüche 4 und 5, in welchem die Fermentation bei einem pH-Wert im Bereich von 5 bis 9 durchgeführt wird.

7. Verfahren gemäß irgendeinem der Ansprüche 4 bis 6, in welchem die AB-021-Antibiotika mittels Filtration und anschließender Verwendung chromatographischer Techniken aus der Fermentationsbrühe isoliert werden.

8. Verfahren gemäß irgendeinem der Ansprüche 4 bis 7, in welchem AB-021 und AB-021b mittels Umkehrphasen-Chromatographie auf einer Kieselgel-Säule unter Verwendung eines linearen Elutions-Gradienten von 30% bis 70% Methanol in entsprechender Mischung mit Wasser, welches 10 mM/I Ammoniummonohydrogenphosphat enthält, isoliert werden.

9. Der Mikroorganismus Streptomyces s p. NCIB 40068.

10. Biologisch reine Kultur von Streptomyces s p. NCIB 40068 oder einer gleichwertigen Mutante davon, welche zur Produktion von AB-021-Antibiotika in isolierbaren Mengen mittels kontrollierter aerober Fermentation in einem wäßrigen Nährmedium, welches assimilierbare Kohlenstoff- und Stickstoff-Quellen sowie anorganische Salze enthält, fähig ist.

11. Fungizide und/oder bakterizide Zusammensetzungen, welche zusammen mit inerten festen oder flüssigen Trägern und gegebenenfalls anderen Zusatzstoffen eine Verbindung, welche ausgewählt ist aus AB-021-Antibiotika, AB-021 und AB-021 b, als Wirkstoff enthalten.
Patentansprüche für folgenden Vertragsstaat : ES

1. Verfahren zur Herstellung des Antibiotikums AB-021a, dadurch gekennzeichnet, daß es umfaßt: eine Kultivierung unter kontrollierten aeroben Fermentationsbedingungen von Streptomyces s p. NCIB 40068 oder einer entsprechenden Mutante davon in einem wäßrigen Nährmedium, welches assimilierbare Kohlenstoff- und Stickstoff-Quellen sowie anorganische Salze enthält, bis eine beträchtliche antibiotische Aktivität erreicht ist,
eine anschließende Gewinnung des besagten Antibiotikums mittels an sich bekannter Verfahren, und eine Abtrennung des Antibiotikums AB-021 a mittels Umkehrphasen-Chromatographie auf einer Kieselgel-Säule unter Verwendung eines linearen Elutions-Gradienten von 30% bis 70% Methanol in entsprechender Mischung mit Wasser, welches 10 mM/1 Ammoniummonohydrogenphosphat enthält, wobei die gewonnene feste Substanz die folgenden Eigenschaften aufweist:
(a) die folgende angenäherte Elementaranalyse: 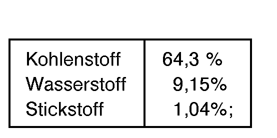
(b) ein Molekulargewicht von etwa 1139;
(c) UV-Absorptionsmaxima von:
0,193 bei 232,8 nm; 2,064 bei 318,4 nm; 2,44 bei 332,6 nm; 2,113 bei 349,6 nm; bei einer Konzentration von 0,025 mg/ml in Acetonitril/Wasser 1:1 (Vol./Vol.);
(d) IR-Absorptionsmaxima bei (cm⁻¹):
3365; 3015; 2923; 2851; 1700; 1634; 1594; 1541; 1431; 1380; 1329; 1262; 1128; 1095; 1056; 1004; 968; 919; 878; 843; 804; 754;
(e) Hauptpeaks in seinem ¹H-NMR Spektrum bei:
δTMS (ppm): 7,18 (d, 1 H); 6,69 (dd, 1 H); 6,59 (dd, 1 H); 6,52 - 6,09 (m, 9H); 5,96 - 5,70 (m, 3H); 5,70 - 5,31 (m, 8H); 4,27 - 4,02 (m, 4H); 4,02 - 3,82 (m, 4 H); 3,82 - 3,58 (m, 4H); 3,30 (t, 1 H); 2,82 (t, 2H); 2,73 - 2,45 (^{*}) (m, 3H); 2,41 - 1,98 (m, 13H); 1,91 (s, 3H); 1,79 - 1,12 (m, 24-25H); 1,00 (d, 3H); 0,95 (d, 3H); 0,89 (d, 3H); 0,85 (d, 3H);
(f) Hauptpeaks in seinem ¹³C-NMR Spektrum bei:
δTMS (ppm): 212,1 (s); 169,9 (s); 138,8 (d); 138,5 (d); 137,0 (d); 136,9 (d); 136,7 (d); 136,1 (d); 135,9 (d); 135,9 (d); 134,0 (d); 133,3 (d); 133,0 (d); 132,9 (d); 132,5 (d); 132,4 (d); 132,0 (d); 131,5 (d); 131,0 (d); 131,0 (d); 129,3 (d); 128,9 (d); 128,0 (d); 126,7 (d); 126,4 (d); 125,3 (d); 75,3 (d); 72,3 (d); 71,2 (d); 70,6 (d); 69,7 (d); 68,9 (d); 68,4 (d); 68,0 (d); 67,1 (d); 67,1 (d); 65,7 (d); 64,2 (d); 64,2 (d); 52,1 (d); 41,5 (d); 39,9 (d); 49,2 (t); 46,2 (t); 46,0 (t); 45,2 (t); 45,2 (t); 44,9 (t); 43,9 (t); 41,2 (t); 40,8 (t); 40,7 (t); 39,2 (t); 38,8 (t); 34,1 (t); 32,8 (t); 32,1 (t); 29,3 (t); 24,1 (t); 22,8 (q); 18,4 (q); 13,2 (q); 10,5 (q); 9,1 (q);
(g) mittels Dünnschicht-Chromatographie (TLC) auf Platten des Typs 60F 254 (Merck-Schuchardt) erhaltene R_{f}-Werte von:
0,47-0,57 in Ethanol : Dioxan : wäßrige Ammoniaklösung (30%): Wasser (8:1:1:1);
0,0 in Methylenchlorid : Methanol (17:3);
mittels Umkehrphasen-Chromatographie auf Platten des Typs RP-18F 254 (Merck-Schuchardt) erhaltene R_{f}-Werte von:
0,29 in Methanol : Acetonitril : 25 mM Natriummonohydrogenphosphat in Wasser (4:4:2);
0,39 in Methanol : Acetonitril : 25 mM Kaliumdihydrogenphosphat + 7 mM Tetramethylammoniumchlorid in Wasser (4:4:2);
0,21 in Methanol : 10 mM wäßrige, mittels Phosphorsäure auf einen pH-Wert von 7,5 eingestellte Lösung von Ammoniummonohydrogenphosphat (8:2);
0,25 in Methanol : Acetonitril : 10 mM wäßrige, mittels Phosphorsäure auf einen pH-Wert von 7,5 eingestellte Lösung von Ammoniummonohydrogenphosphat (4:4:2);
(h) eine Retentionszeit (Rₜ) von 8 Minuten bei der UmkehrphasenHPLC auf einer Hibar Li-Chro-CART@ Li-Chrosorb RP-18 Säule (Vorsäule: Guard Pak@ RCSS C 18) unter Verwendung einer Elutionsmischung aus Methanol : Acetonitril : 10 mM wäßrige Lösung von Ammoniummonohydrogenphosphat(1:1:1) bei einer Strömungsgeschwindigkeit von 0,8 ml/min sowie 40 ° C;
(i) eine gute Löslichkeit in Dimethylsulfoxid und in Mischungen aus Ethanol/Wasser (1:1 Vol./Vol.) oder Methanol/Wasser (1:1 Vol./Vol.), sowie schlechte Löslichkeit in Wasser und recht gute Löslichkeit in Ethanol und Methanol.

2. Verfahren zur Herstellung des Antibiotikums AB-021 b, dadurch gekennzeichnet, daß es umfaßt:
eine Kultivierung unter kontrollierten aeroben Fermentationsbedingungen von Streptomyces s p. NCIB 40068 oder einer entsprechenden Mutante davon in einem wäßrigen Nährmedium, welches assimilierbare Kohlenstoff- und Stickstoff-Quellen sowie anorganische Salze enthält, bis eine beträchtliche antibiotische Aktivität erreicht ist,
eine anschließende Gewinnung des besagten Antibiotikums mittels an sich bekannter Verfahren, und eine Abtrennung des Antibiotikums AB-021 b mittels Umkehrphasen-Chromatographie auf einer Kieselgel-Säule unter Verwendung eines linearen Elutions-Gradienten von 30% bis 70% Methanol in entsprechender Mischung mit Wasser, welches 10 mM/1 Ammoniummonohydrogenphosphat enthält,
wobei die gewonnene feste Substanz die folgenden Eigenschaften aufweist:
((^{*}) der Peak des DMSO-d6 erscheint ebenfalls innerhalb dieses Bereiches);
(a) die folgende angenäherte Elementaranalyse: 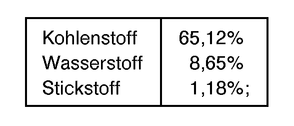
(b) ein Molekulargewicht von etwa 1165;
(c) UV-Absorptionsmaxima von:
0,90 bei 369,0 nm; 2,01 bei 350,0 nm; 2,21 bei 332,6 nm; 1,49 bei 318,0 nm; bei einer Konzentration von 0,02 mg/ml in Acetonitril/Wasser 1:1 (Vol./Vol.);
(d) IR-Absorptionsmaxima bei (cm⁻¹):
3902, 3853, 3747, 3375, 3013, 2921, 2853, 2757, 1895, 1699, 1669, 1645, 1576, 1540, 1430, 1378, 1324, 1261, 1160, 1094, 1059, 1004, 969, 919, 878, 843, 803, 779, 697;
(e) Hauptpeaks in seinem ¹H-NMR Spektrum bei:
δTMS (ppm): 7,21 (d, 1 H); 6,75 (dd, 1 H); 6,63 (dd, 1 H); 6,57 - 6,07 (m, 11 H); 5,93 - 5,70 (m, 3H); 5,70 - 5,33 (m, 8H); 4,25 - 4,20 (m, 4H); 4,02 - 3,83 (m, 4 H); 3,83 - 3,59 (m, 4H); 3,29 (t, 1 H); 2,82 (t, 2H); 2,72 - 2,44 (^{*}) (m, 6-7H); 2,41 -2,00 (m, 14H); 1,92 (s, 3H); 1,80 - 1,14 (m, 21 H); 1,00 (d, 3H); 0,95 (d, 3H); 0,84 (d, 3H);
(f) Hauptpeaks in seinem ¹³C-NMR Spektrum bei:
δTMS (ppm): 212,0 (s); 169,2 (s); 139,1 (d); 138,1 (d); 137,1 (d); 136,7 (d); 136,2 (d); 135,8 (d); 135,8 (d); 135,1 (d); 133,8 (d); 133,1 (d); 132,8 (d); 132,8 (d); 132,6 (d); 132,3 (d); 132,2 (d); 132,1 (d); 131,8 (d); 131,2 (d); 130,9 (d); 130,8 (d); 129,2 (d); 128,7 (d); 127,9 (d); 126,4 (d); 126,2 (d); 125,2 (d); 75,4 (d); 72,6 (d); 71,0 (d); 70,6 (d); 69,6 (d); 68,9 (d); 68,4 (d); 68,0 (d); 67,3 (d); 67,2 (d); 65,6 (d); 64,4 (d); 64,4 (d); 51,9 (d); 41,3 (d); 39,6 (d); 49,2 (t); 45,8 (t); 45,7 (t); 45,0 (t); 44,9 (t); 44,7 (t); 43,6 (t); 41,1 (t); 40,7 (t); 40,5 (t); 39,0 (t); 38,7 (t); 33,9 (t); 32,5 (t); 31,9 (t); 29,0 (t); 23,9 (t); 18,1 (q); 12,8 (q); 10,4 (q); 8,7 (q);
(g) mittels Dünnschicht-Chromatographie (TLC) auf Platten des Typs 60F 254 (Merck-Schuchardt) erhaltene R_{f}-Werte von:
0,54 in Ethanol : Dioxan : wäßrige Ammoniaklösung (30%) : Wasser (8:1:1:1);
0,00 in Methylenchlorid : Methanol (17:3);
mittels Umkehrphasen-Chromatographie auf Platten des Typs RP-18F 254 (Merck-Schuchardt) erhaltene R_{f}-Werte von:
0,27 in Methanol : Acetonitril : 25 mM Natriummonohydrogenphosphat in Wasser (4:4:2);
0,35 in Methanol : Acetonitril : 25 mM Kaliumdihydrogenphosphat + 7 mM Tetramethylammoniumchlorid in Wasser (4:4:2);
0,12 in Methanol : 10 mM wäßrige, mittels Phosphorsäure auf einen pH-Wert von 7,5 eingestellte Lösung von Ammoniummonohydrogenphosphat (8:2);
0,24 in Methanol : Acetonitril : 10 mM wäßrige, mittels Phosphorsäure auf einen pH-Wert von 7,5 eingestellte Lösung von Ammoniummonohydrogenphosphat (4:4:2);
(h) eine Retentionszeit (Rₜ) von 11,8 Minuten bei der UmkehrphasenHPLC auf einer Hibar Li-ChroCART® Li-Chrosorb® RP-18 Säule (Vorsäule: Guard Pak° RCSS C18) unter Verwendung einer Elutionsmischung aus Methanol : Acetonitril : 10 mM wäßrige Lösung von Ammoniummonohydrogenphosphat(1:1:1) bei einer Strömungsgeschwindigkeit von 0,8 ml/min sowie 40 ° C.

3. Verfahren zur Herstellung der AB-021-Antibiotika, welche im wesentlichen das Antibiotikum AB-021a und das Antibiotikum AB-021 gemäß der Definitionen in den Ansprüchen 1 und 2 beinhalten, dadurch gekennzeichnet, daß es umfaßt:
eine Kultivierung unter kontrollierten aeroben Fermentationsbedingungen von Streptomyces s p. NCIB 40068 oder einer entsprechenden Mutante davon in einem wäßrigen Nährmedium, welches assimilierbare Kohlenstoff- und Stickstoff-Quellen sowie anorganische Salze enthält, bis eine beträchtliche antibiotische Aktivität erreicht ist, und
eine anschließende Gewinnung der besagten Antibiotika mittels an sich bekannter Verfahren.

4. Verfahren gemäß irgendeinem der Ansprüche 1 bis 3, dadurch gekennzeichnet, daß die Fermentation bei einer Temperatur von 20 ° C bis 35 ° C durchgeführt wird.
((^{*}) der Peak des DMSO-d6 erscheint ebenfalls innerhalb dieses Bereiches);

5. Verfahren gemäß irgendeinem der Ansprüche 1 bis 4, dadurch gekennzeichnet, daß die Fermentation bei einem pH-Wert im Bereich von 5 bis 9 durchgeführt wird.

6. Verfahren gemäß irgendeinem der Ansprüche 1 bis 5, dadurch gekennzeichnet, daß die AB-021-Antibiotika mittels Filtration und anschließender Verwendung chromatographischer Techniken aus der Fermentationsbrühe isoliert werden.

## Revendications (Revendications pour l'(les) Etat(s) contractant(s) suivant(s) : AT, BE, CH, DE, FR, GB, LI, NL, SE)

1. Antibiotique AB-021 a consistant en une substance solide caractérisée par :
(a) l'analyse élémentaire approximative suivante: 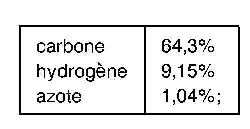
(b) un poids moléculaire d'environ 1139;
(c) des maxima d'absorbance dans l'UV de:
0,193 à 232,8 nm; 2,064 à 318,4 nm; 2,44 à 332,6 nm; 2,113 à 349,6 nm, à une concentration de 0,025 mg/ml dans un mélange acétonitrile/eau 1/1 (v/v);
(d) des maxima d'absorbance dans l'infrarouge à (cm⁻¹):
3365; 3015; 2923; 2851; 1700; 1634; 1594; 1541; 1431; 1380; 1329; 1262; 1128; 1095; 1056; 1004; 968; 919; 878; 843; 804; 754;
(e) des pics principaux dans son spectre ¹H-RMN à:
₅TMS (ppm); 7,18 (d, 1 H); 6,69 (dd, 1 H); 6,59 (dd, 1 H); 6,52 - 6,09 (m, 9H); 5,96 - 5,70 (m, 3H); 5,70 - 5,31 (m, 8H); 4,27 - 4,02 (m, 4H); 4,02 - 3,82 (m, 4H); 3,82 - 3,58 (m, 4H); 3,30 (t, 1 H); 2,82 (t, 2H); 2,73 - 2,45 (^{*}) (m, 3H); 2,41 - 1,98 (m, 13H); 1,91 (s, 3H); 1,79 - 1,12 (m, 24-25H); 1,00 (d, 3H); 0,95 (d, 3H); 0,89 (d, 3H); 0,85 (d, 3H);
(f) des pics principaux dans son spectre ¹³C-RMN à:
₅TMS (ppm): 212,1 (s); 169,9 (s); 138,8 (d); 138,5 (d); 137,0 (d); 136,9 (d); 136,7 (d); 136,1 (d); 135,9
(d); 135,9 (d); 134,0 (d); 133,3 (d); 133,0 (d); 132,9 (d); 132,5 (d); 132,4 (d); 132,0 (d); 131,5 (d); 131,0 (d); 131,0 (d); 129,3 (d); 128,9 (d); 128,0 (d); 126,7 (d); 126,4 (d); 125,3 (d); 75,3 (d); 72,3 (d); 71,2 (d); 70,6 (d); 69,7 (d); 68,9 (d); 68,4 (d); 68,0 (d); 67,1 (d); 67,1 (d); 65,7 (d); 64,2 (d); 64,2 (d); 52,1 (d); 41,5 (d); 39,9 (d); 49,2 (t); 46,2 (t); 46,0 (t); 45,2 (t); 45,2 (t); 44,9 (t); 43,9 (t); 41,2 (t); 40,8 (t); 40,7 (t); 39,2 (t); 38,8 (t); 34,1 (t); 32,8 (t); 32,1 (t); 29,3 (t); 24,1 (t); 22,8 (q); 18,4 (q); 13,2 (q); 10,5 (q); 9,1 (q);
(g) des valeurs R_{f} obtenues par chromatographie sur couche mince (TLC) sur des plaques de type 60F 254 (Merck Schuchardt):
0,47-0,57 dans un mélange éthanol/dioxane/solution aqueuse d'ammoniaque (à 30%)/eau (8/1/1/1);
0,0 dans un mélange chlorure de méthylène/méthanol (17/3);
des valeurs R_{f} obtenues par chromatographie en phase inverse sur des plaques de type RP-18F 254 (Merck-Schuchardt):
0,29 dans un mélange méthanol/acétonitrile/25 mM de monohydrogénophosphate de sodium dans l'eau (4/4/2);
0,39 dans un mélange méthanol/acétonitrile/25 mM de dihydrogénophosphate de potassium + 7 mM de chlorure de tétraméthyl-ammonium dans l'eau (4/4/2);
0,21 dans un mélange méthanol/solution aqueuse de monohydrogénophosphate d'ammonium à 10 mM dont le pH est ajusté à 7,5 avec de l'acide phosphorique (8/2);
0,25 dans un mélange méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) de 8 minutes en HPLC en phase inverse sur une colonne Hibar Li-ChroCART@ Li-Chrosorb RP-18, (précolonne: Guard Pak@ RCSS C 18) lorsqu'elle est éluée avec un mélange méthanol/acétonitrile/solution aqueuse de monohydrogénophosphate d'ammonium à 10 mM (1/1/1) à un débit de 0,8 ml/minute et à 40°C;
((^{*}) dans cet intervalle, le pic du DMSO-d6 apparaît également);
(i) une bonne solubilité dans le diméthylsulfoxyde et dans les mélanges éthanol/eau (1/1 V/V) ou méthanol/eau (1/1 V/V), une faible solubilité dans l'eau et une assez bonne solubilité dans l'éthanol et le méthanol.

2. Antibiotique AB-021 b, consistant en une substance solide caractérisée par:
(a) l'analyse élémentaire approximative suivante: 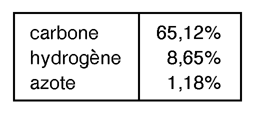
(b) un poids moléculaire d'environ 1165;
(c) des maxima d'absorbance dans l'UV de:
0,90 à 369,0 nm; 2,01 à 350,0 nm; 2,21 à 332,6 nm; 1,49 à 318,0 nm; à une concentration de 0,02 mg/ml dans un mélange acétonitrile/eau 1/1 (V/V);
(d) des maxima d'absorbance dans l'infrarouge à (cm⁻¹):
3902, 3853, 3747, 3375, 3013, 2921, 2853, 2757, 1895, 1699, 1669, 1645, 1576, 1540, 1430, 1378, 1324, 1261, 1160, 1094, 1059, 1004, 969, 919, 878, 843, 803, 779, 697;
(e) des pics principaux dans son spectre ¹H-RMN à:
₅TMS (ppm): 7,21 (d, 1 H); 6,75 (dd, 1 H); 6,63 (dd, 1 H); 6,57 - 6,07 (m, 11 H); 5,93 - 5,70 (m, 3H); 5,70 - 5,33 (m, 8H); 4,25 - 4,20 (m, 4H); 4,02 - 3,83 (m, 4H); 3,83 - 3,59 (m, 4H); 3,29 (t, 1 H); 2,82 (t, 2H); 2,72 - 2,44 (^{*}) (m, 6-7H); 2,41 - 2,00 (m, 14H); 1,92 (s, 3H); 1,80 - 1,14 (m, 21 H); 1,00 (d, 3H); 0,95 (d, 3H); 0,84 (d, 3H).
(f) des pics principaux dans son spectre ¹³C-RMN à:
₅TMS (ppm): 212,0 (s); 169,2 (s); 139,1 (d); 138,1 (d); 137,1 (d); 136,7 (d); 136,2 (d); 135,8 (d); 135,8 (d); 135,1 (d); 133,8 (d); 133,1 (d); 132,8 (d); 132,8 (d); 132,6 (d); 132,3 (d); 132,2 (d); 132,1 (d); 131,8 (d); 131,2 (d); 130,9 (d); 130,8 (d); 129,2 (d); 128,7 (d); 127,9 (d); 126,4 (d); 126,2 (d); 125,2 (d); 75,4 (d); 72,6 (d); 71,0 (d); 70,6 (d); 69,6 (d); 68,9 (d); 68,4 (d); 68,0 (d); 67,3 (d); 67,2 (d); 65,6 (d); 64,4 (d); 64,4 (d); 51,9 (d); 41,3 (d); 39,6 (d); 49,2 (t); 45,8 (t); 45,7 (t); 45,0 (t); 44,9 (t); 44,7 (t); 43,6 (t); 41,1 (t); 40,7 (t); 40,5 (t); 39,0 (t); 38,7 (t); 33,9 (t); 32,5 (t); 31,9 (t); 29,0 (t); 23,9 (t); 18,1 (q); 12,8 (q); 10,4 (q); 8,7 (q);
(g) des valeurs R_{f} obtenues par chromatographie sur couche mince (TLC) sur des plaques de type 60F 254 (Merck-Schuchardt):
0,54 dans un mélange éthanol/dioxane/solution aqueuse d'ammoniaque (30%)/eau (8/1/1/1);
0,00 dans un mélange chlorure de méthylène/méthanol (17/3);
des valeurs R_{f} obtenues par chromatographie en phase inverse sur des plaques de type RP-18F 254 (Merck-Schuchardt):
0,27 dans un mélange méthanol/acétonitrile/25 mM de monohydrogénophosphate de sodium dans l'eau (4/4/2);
0,35 dans un mélange méthanol/acétonitrile/25 mM de dihydrogénophosphate de potassium dans l'eau + 7 mM de chlorure de tétraméthylammonium dans l'eau (4/4/2);
0,12 dans un mélange méthanol/solution aqueuse de monohydrogénophosphate d'ammonium à 10 mM dont le pH est ajusté à 7,5 avec de l'acide phosphorique (8/2);
0,24 dans un mélange méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) de 11,8 minutes en HPLC en phase inverse sur une colonne Hibar Li-ChroCART@ Li-Chrosorb@ RP18, (précolonne: Guard Pak@ RCSS C18) lorsqu'elle est éluée avec un mélange méthanol/acétonitrile/solution aqueuse à 10 mM de monohydrogénophosphate d'ammonium (1/1/1) à un débit de 0,8 ml/minute et à 40 ° C.

3. Antibiotiques AB-021, susceptibles d'être obtenus au moyen d'une culture contrôlée dans les conditions aérobies de Streptomyces s.p. NCIB 40068 ou d'un mutant équivalent en dérivant, dans un milieu de culture nutritif aqueux contenant des sources de carbone, d'azote et de sels inorganiques, lesdits antibiotiques étant sensiblement composés de l'antibiotique AB-021 a et de l'antibiotique AB-021 b tels que définis dans les revendications 1 et 2.
((^{*}) dans cet intervalle le pic du DMSO-d6 apparaît également);

4. Procédé de préparation des antibiotiques AB-021 comprenant la culture de Streptomyces s p. NCIB 40068 ou d'un mutant correspondant en dérivant, dans les conditions de fermentation aérobie contrôlées dans un milieu nutritif aqueux contenant des sources assimilables de carbone et d'azote ainsi que des sels inorganiques jusqu'à ce qu'une activité antibiotique substantielle soit obtenue, la récupération ultérieure desdits antibiotiques au moyen de méthodes connues per se et éventuellement la séparation des composants principaux, désignés AB-021 a et AB-021 b, tels que définis dans les revendications 1 et 2.

5. Procédé selon la revendication 4, dans lequel la fermentation est mise en oeuvre à une température de 20 à 35 _{°} C.

6. Procédé selon l'une quelconque des revendications 4 et 5, dans lequel la fermentation est mise en oeuvre à un pH compris dans un intervalle de 5 à 9.

7. Procédé selon l'une quelconque des revendications 4 à 6, dans lequel les antibiotiques AB-021 sont isolés à partir du bouillon de fermentation par filtration puis par utilisation des techniques chromatographiques.

8. Procédé selon l'une quelconque des revendications 4 à 7, dans lequel AB-021 a et AB-021 b sont isolés par chromatographie en phase inverse sur une colonne de gel de silice en utilisant un gradient d'élution linéaire de 30 à 70% de méthanol dans le mélange correspondant avec de l'eau contenant 10 mM/1 de monohydrogénophosphate d'ammonium.

9. Microorganisme Streptomyces s p. NCIB 40068.

10. La culture biologiquement pure de Streptomyces sp. NCIB 40068 ou d'un mutant équivalent en dérivant, capable de produire les antibiotiques AB-021, en des quantités susceptibles d'être isolées, au moyen d'une fermentation aérobie contrôlée dans un milieu nutritif aqueux comprenant des sources assimilables de carbone et d'azote ainsi que des sels inorganiques.

11. Compositions fongicides et/ou bactéricides contenant en tant que principe actif, un composé sélectionné parmi les antibiotiques AB-021, AB-021 a et AB-021 b, ainsi que des supports liquides ou solides inertes et éventuellement d'autres additifs.

## Revendications (Revendications pour l'(les) Etat(s) contractant(s) suivant(s) : suivant : ES)

1. Un procédé pour la préparation de l'antibiotique AB-021 a, caractérisé en ce qu'il comprend la culture de Streptomyces s p. NCIB 40068 ou d'un mutant correspondant en dérivant, dans des conditions de fermentation aérobie contrôlées dans un milieu nutritif aqueux contenant des sources assimilables de carbone et d'azote ainsi que des sels inorganiques jusqu'à ce qu'une activité antibiotique substantielle soit obtenue, la récupération ultérieure dudit antibiotique au moyen de méthodes connues per se, et la séparation de l'antibiotique AB-021 a par chromatographie en phase inverse sur une colonne de gel de silice utilisant un gradient d'élution linéaire de 30% à 70% de méthanol dans le mélange correspondant avec de l'eau contenant 10 mM/I de monohydrogénophosphate d'ammonium, la substance solide récupérée présentant les propriétés suivantes:
(a) l'analyse élémentaire approximative suivante: 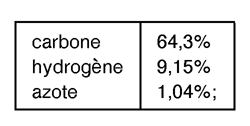
(b) un poids moléculaire d'environ 1139;
(c) des maxima d'absorbance dans l'UV de:
0,193 à 232,8 nm; 2,064 à 318,4 nm; 2,44 à 332,6 nm; 2,113 à 349,6 nm, à une concentration de 0,025 mg/ml dans un mélange acétonitrile/eau 1/1 (v/v);
(d) des maxima d'absorbance dans l'infrarouge à (cm-'):
3365; 3015; 2923; 2851; 1700; 1634; 1594: 1541; 1431; 1380; 1329; 1262; 1128; 1095; 1056; 1004; 968; 919; 878; 843; 804; 754;
(e) des pics principaux dans son spectre ¹H-RMN à:
₅TMS (ppm): 7,18 (d, 1 H); 6,69 (dd, 1 H); 6,59 (dd, 1 H); 6,52 - 6,09 (m, 9H); 5,96 - 5,70 (m, 3H); 5,70 - 5,31 (m, 8H); 4,27 - 4,02 (m, 4H); 4,02 - 3,82 (m, 4H); 3,82 - 3,58 (m, 4H); 3,30 (t, 1 H); 2,82 (t, 2H); 2,73 - 2,45 (^{*})(m, 3H); 2,41 - 1,98 (m, 13H); 1,91 (s, 3H); 1,79 - 1,12 (m, 24-25H); 1,00 (d, 3H); 0,95 (d, 3H); 0,89 (d, 3H); 0,85 (d, 3H);
(f) des pics principaux dans son spectre ¹³C-RMN à:
δTMS (ppm): 212,1 (s); 169,9 (s); 138,8 (d); 138,5 (d); 137,0 (d); 136,9 (d); 136,7 (d); 136,1 (d); 135,9 (d); 135,9 (d); 134,0 (d); 133,3 (d); 133,0 (d); 132,9 (d); 132,5 (d); 132,4 (d); 132,0 (d); 131,5 (d); 131,0 (d); 131,0 (d); 129,3 (d); 128,9 (d); 128,0 (d); 126,7 (d); 126,4 (d); 125,3 (d); 75,3 (d); 72,3 (d); 71,2 (d); 70,6 (d); 69,7 (d); 68,9 (d); 68,4 (d); 68,0 (d); 67,1 (d); 67,1 (d); 65,7 (d); 64,2 (d); 64,2 (d); 52,1 (d); 41,5 (d); 39,9 (d); 49,2 (t); 46,2 (t); 46,0 (t); 45,2 (t); 45,2 (t); 44,9 (t); 43,9 (t); 41,2 (t); 40,8 (t); 40,7 (t); 39,2 (t); 38,8 (t); 34,1 (t); 32,8 (t); 32,1 (t); 29,3 (t); 24,1 (t); 22,8 (q); 18,4 (q); 13,2 (q); 10,5 (q); 9,1 (q);
(g) des valeurs R_{f} obtenues par chromatographie sur couche mince (TLC) sur des plaques de type 60F 254 (Merck Schuchardt):
0,47-0,57 dans un mélange éthanol/dioxane/solution aqueuse d'ammoniaque (30%)/eau (8/1/1/1);
0,0 dans un mélange chlorure de méthylène/méthanol (17/3);
des valeurs R_{f} obtenues par chromatographie en phase inverse sur des plaques de type RP-18F 254 (Merck-Schuchardt):
0,29 dans un mélange méthanol/acétonitrile/25 mM de monohydrogéno-phosphate de sodium dans l'eau (4/4/2);
0,39 dans un mélange méthanol/acétonitrile/25 mM de dihydrogéno-phosphate de potassium + 7 mM de chlorure de tétraméthyl-ammonium dans l'eau (4/4/2);
0,21 dans un mélange méthanol/solution aqueuse de monohydrogéno-phosphate d'ammonium à 10 mM dont le pH est ajusté à 7,5 avec de l'acide phosphorique (8/2);
0,25 dans un mélange méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) de 8 minutes en HPLC en phase inverse sur une colonne Hibar Li-ChroCART@ Li-Chrosorb RP-18, (précolonne: Guard Pak@ RCSS C 18) lorsqu'elle est éluée avec un mélange méthanol/acétonitrile/solution aqueuse de monohydrogénophosphate d'ammonium à 10 mM (1/1/1) à un débit de 0,8 ml/minute et à 40°C;
(i) une bonne solubilité dans le diméthylsulfoxyde et dans les mélanges éthanol/eau (1/1 V/V) ou méthanol/eau (1/1 V/V), une faible solubilité dans l'eau et une assez bonne solubilité dans l'éthanol et le méthanol.

2. Un procédé pour la préparation de l'antibiotique AB-021b, caractérisé en ce qu'il comprend la culture de Streptomyces s p. NCIB 40068 ou d'un mutant correspondant en dérivant, dans des conditions de fermentation aérobie contrôlée dans un milieu nutritif aqueux contenant des sources assimilables de carbone et d'azote ainsi que des sels inorganiques jusqu'à ce qu'une activité antibiotique substantielle soit obtenue, la récupération ultérieure dudit antibiotique au moyen de méthodes connues per se, et la séparation de l'antibiotique AB-021 par chromatographie en phase inverse sur une colonne de gel de silice utilisant un gradient d'élution linéaire de 30% à 70% de méthanol dans le mélange correspondant avec de l'eau contenant 10 mM/I de monohydrogénophosphate d'ammonium, la substance solide récupérée présentant les propriétés suivantes:
(a) l'analyse élémentaire approximative suivante: 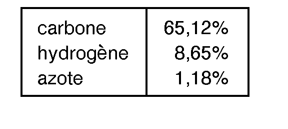
(b) un poids moléculaire d'environ 1165;
(c) des maxima d'absorbance dans l'UV de:
0,90 à 369,0 nm; 2,01 à 350,0 nm; 2,21 à 332,6 nm; 1,49 à 318,0 nm; à une concentration de 0,02 mg/ml dans un mélange acétonitrile/eau 1/1 (V/V);
((^{*}) dans cet intervalle, le pic du DMSO-d6 apparaît également);
(d) des maxima d'absorbance dans l'infrarouge à (cm⁻¹):
3902, 3853, 3747, 3375, 3013, 2921, 2853, 2757, 1895, 1699, 1669, 1645, 1576, 1540, 1430, 1378, 1324, 1261, 1160, 1094, 1059, 1004, 969, 919, 878, 843, 803, 779, 697;
(e) des pics principaux dans son spectre ¹H-RMN à:
₅TMS (ppm): 7,21 (d, 1 H); 6,75 (dd, 1 H); 6,63 (dd, 1 H); 6,57 - 6,07 (m, 11 H); 5,93 - 5,70 (m, 3H); 5,70 - 5,33 (m, 8H); 4,25 - 4,20 (m, 4H); 4,02 - 3,83 (m, 4H); 3,83 - 3,59 (m, 4H); 3,29 (t, 1 H); 2,82 (t, 2H); 2,72 - 2,44 (^{*}) (m, 6-7H); 2,41 - 2,00 (m, 14H); 1,92 (s, 3H); 1,80 - 1,14 (m, 21 H); 1,00 (d, 3H); 0,95 (d, 3H); 0,84 (d, 3H).
(f) des pics principaux dans son spectre ¹³C-RMN à:
₅TMS (ppm): 212,0 (s); 169,2 (s); 139,1 (d); 138,1 (d); 137,1 (d); 136,7 (d); 136,2 (d); 135,8 (d); 135,8 (d); 135,1 (d); 133,8 (d); 133,1 (d); 132,8 (d); 132,8 (d); 132,6 (d); 132,3 (d); 132,2 (d); 132,1 (d); 131,8 (d); 131,2 (d); 130,9 (d); 130,8 (d); 129,2 (d); 128,7 (d); 127,9 (d); 126,4 (d); 126,2 (d); 125,2 (d); 75,4 (d); 72,6 (d); 71,0 (d); 70,6 (d); 69,6 (d); 68,9 (d); 68,4 (d); 68,0 (d); 67,3 (d); 67,2 (d); 65,6 (d); 64,4 (d); 64,4 (d); 51,9 (d); 41,3 (d); 39,6 (d); 49,2 (t); 45,8 (t); 45,7 (t); 45,0 (t); 44,9 (t); 44,7 (t); 43,6 (t); 41,1 (t); 40,7 (t); 40,5 (t); 39,0 (t); 38,7 (t); 33,9 (t); 32,5 (t); 31,9 (t); 29,0 (t); 23,9 (t); 18,1 (q); 12,8 (q); 10,4 (q); 8,7 (q);
(g) des valeurs R_{f} obtenues par chromatographie sur couche mince (TLC) sur des plaques de type 60F 254 (Merck-Schuchardt):
0,54 dans un mélange éthanol/dioxane/solution aqueuse d'ammoniaque (30%)/eau (8/1/1/1);
0,00 dans un mélange chlorure de méthylène/méthanol (17/3);
des valeurs R_{f} obtenues par chromatographie en phase inverse sur des plaques de type RP-18F 254 (Merck-Schuchardt):
0,27 dans un mélange méthanol/acétonitrile/25 mM de monohydrogéno-phosphate de sodium dans l'eau (4/4/2);
0,35 dans un mélange méthanol/acétonitrile/25 mM de dihydrogéno-phosphate de potassium dans l'eau + 7 mM de chlorure de tétraméthylammonium dans l'eau (4/4/2);
0,12 dans un mélange méthanol/solution aqueuse de monohydrogéno-phosphate d'ammonium à 10 mM dont le pH est ajusté à 7,5 avec de l'acide phosphorique (8/2);
0,24 dans un mélange méthanol/acétonitrile/solution aqueuse de monohydrogénophosphate d'ammonium à 10 mM dont le pH est ajusté à 7,5 avec de l'acide phosphorique (4/4/2);
(h) un temps de rétention (Rₜ) de 11,8 minutes en HPLC en phase inverse sur une colonne Hibar Li-ChroCART@ Li-Chrosorb@ RP18, (précolonne: Guard Pak@ RCSS C18) lorsqu'elle est éluée avec un mélange méthanol/acétonitrile/solution aqueuse à 10 mM de monohydrogénophosphate d'ammonium (1/1/1) à un débit de 0,8 ml/minute et à 40 ° C.

3. Procédé de préparation d'antibiotiques AB-021, ces antibiotiques étant sensiblement composés de l'antibiotique AB-021 a et de l'antibiotique AB-021 b, tels que définis dans les revendications 1 et 2, caractérisé en ce qu'il comprend la culture de Streptomyces s.p. NCIB 40068 ou d'un mutant équivalent en dérivant, dans des conditions de fermentation aérobie contrôlée dans un milieu de culture nutritif aqueux contenant des sources assimilables de carbone et d'azote ainsi que de sels inorganiques, jusqu'à ce qu'une activité antibiotique substantielle soit obtenue et la récupération ultérieure desdits antibiotiques au moyen de méthodes connues per se.

4. Procédé selon l'une des revendications 1 à 3, caractérisé en que la fermentation est mise en oeuvre à une température de 20 à 35 ° C.

5. Procédé selon l'une quelconque des revendications 1 à 4, caractérisé en ce que la fermentation est mise en oeuvre à un pH compris dans un intervalle de 5 à 9.

6. Procédé selon l'une quelconque des revendications 1 à 5, caractérisé en ce que les antibiotiques AB-021 sont isolés à partir du bouillon de fermentation par filtration puis par utilisation des techniques chromatographiques.
((^{*}) dans cet intervalle le pic du DMSO-d6 apparaît également);
